(19) Europäisches Patentamt
European Patent Office
Office européen des brevets

(11) Veröffentlichungsnummer : **0 246 187 B1**

(12) **EUROPÄISCHE PATENTSCHRIFT**

(45) Veröffentlichungstag der Patentschrift :
**05.02.92 Patentblatt 92/06**

(51) Int. Cl.$^5$ : **C07D 499/00,** A61K 31/43,
// C07D401/12

(21) Anmeldenummer : **87810278.9**

(22) Anmeldetag : **30.04.87**

(54) **2-Pyridyl-penem-Verbindungen.**

Die Akte enthält technische Angaben, die nach dem Eingang der Anmeldung eingereicht wurden und die nicht in dieser Patentschrift enthalten sind.

(30) Priorität : **06.05.86 CH 1846/86**

(43) Veröffentlichungstag der Anmeldung :
**19.11.87 Patentblatt 87/47**

(45) Bekanntmachung des Hinweises auf die Patenterteilung :
**05.02.92 Patentblatt 92/06**

(84) Benannte Vertragsstaaten :
**AT BE CH DE ES FR GB GR IT LI LU NL SE**

(56) Entgegenhaltungen :
**EP-A- 0 002 210**
**EP-A- 0 070 204**
**GB-A- 2 042 515**

(73) Patentinhaber : **CIBA-GEIGY AG**
**Klybeckstrasse 141**
**CH-4002 Basel (CH)**

(72) Erfinder : **Lang, Marc, Dr.**
**Rue des Ormes 6**
**F-68170 Rixheim (FR)**

EP 0 246 187 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Aus der Europäischen Patentanmeldung Nr. 2210 sind 2-Penem-3-carbonsäure-Verbindungen bekannt, welche in Position 2 des Penemringes durch einen 2-Pyridyl-Rest substituiert sind. Die Verbindungen weisen eine antibakterielle Wirksamkeit auf.

Der vorliegenden Erfindung liegt die Aufgabe zugrunde, neue Penem-Verbindungen bereitzustellen, welche gegenüber den aus dem genannten Stand der Technik bekannten Verbindungen verbesserte pharmakologische Eigenschaften besitzen.

Die Erfindung betrifft insbesondere 2-Pyridyl-penem-Verbindungen der Formel

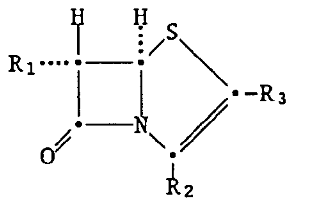

(I),

worin $R_1$ Hydroxymethyl oder 1-Hydroxyäthyl ist, $R_2$ Carboxyl oder unter physiologischen Bedingungen spaltbares verestertes Carboxyl bedeutet; und $R_3$ 3-Pyridyl oder durch Hydroxy, Niederalkoxy, Aminoniederalkoxy, Niederalkanoyloxy, Halogen, Mercapto, Niederalkylthio, Aminoniederalkylthio, Phenylthio, Niederalkyl, Hydroxyniederalkyl, Niederalkanoyloxyniederalkyl, Carboxyniederalkyl, Niederalkoxycarbonylniederalkyl, durch Cyano oder Amino substituiertes Niederalkoxycarbonylniederalkyl, Carbamoylniederalkyl, Carbamoyloxyniederalkyl, Cyanoniederalkyl, Halogenniederalkyl, Aminoniederalkyl, Niederalkylaminoniederalkyl, Diniederalkylaminoniederalkyl, Niederalkanoylaminoniederalkyl, Amino-carboxyniederalkyl, Sulfoniederalkyl, Amino, Niederalkylamino, Diniederalkylamino, Niederalkanoylamino, Carboxyl, Niederalkoxycarbonyl, durch Cyano oder Amino substituiertes Niederalkoxycarbonyl, Carbamoyl, Cyano, Sulfo, Sulfamoyl, Oxido und/oder gegebenenfalls durch Niederalkyl, Niederalkoxy und/oder Halogen substituiertes Phenyl substituiertes 3-Pyridyl darstellt, worin mit "nieder" bezeichnete Reste 1 bis 7 Kohlenstoffatome enthalten, Salze von Verbindungen der Formel I, die eine salzbildende Gruppe aufweisen, Verfahren zur Herstellung von Verbindungen der Formel I, pharmazeutische Präparate, die solche Verbindungen enthalten, und ihre Verwendung zur Herstellung von pharmazeutischen Präparaten.

Die vor- und nachstehend verwendeten Definitionen haben im Rahmen der vorliegenden Beschreibung vorzugsweise die folgenden Bedeutungen:

Unter physiologischen Bedingungen spaltbare (d.h. metabolisierbare) veresterte Carboxylgruppen $R_2$ sind aus der Cephalosporin-, Penicillin- und Penemchemie bekannt. Geeignete Gruppen sind in erster Linie Acyloxymethoxycarbonylgruppen, worin Acyl z.B. den Rest einer organischen Carbonsäure, in erster Linie einer gegebenenfalls substituierten Niederalkancarbonsäure, bedeutet oder worin Acyloxymethyl den Rest eines Lactons bildet. Solche Gruppen sind z.B. Niederalklanoyloxymethoxycarbonyl, Aminoniederalkanoyloxymethoxycarbonyl, insbesondere $\alpha$-Aminoniederalkanoyloxymethoxycarbonyl, und 4-Crotonolactonyl. Weitere unter physiologischen Bedingungen spaltbare veresterte Carboxylgruppen $R_2$ sind z.B. 5-Indanyloxycarbonyl, Phthalidyloxycarbonyl, 1-Niederalkoxycarbonyloxyniederalkoxycarbonyl, 1-Niederalkoxyniederalkoxycarbonyl oder auch 2-Oxo-1,3-dioxolen-4-ylmethoxycarbonyl, welches in 5-Stellung des Dioxolenringes gegebenenfalls durch Niederalkyl oder Phenyl substituiert ist.

Der Pyridyl-Rest in Verbindungen der Formel I kann mono- oder auch poly-, in erster Linie mono- oder disubstituiert, sein.

Die Substituenten sind insbesondere an Kohlenstoffatomen des Pyridyl-Restes $R_3$ gebunden, können aber auch, wie Insbesondere gegebenenfalls wie angegeben substituiertes Niederalkyl oder Oxido, am Pyridyl-Stickstoffatom gebunden sein.

In der vorliegenden Beschreibung bedeutet der im Zusammenhang mit Definitionen von Gruppen bzw. Verbindungen verwendete Ausdruck "nieder", dass die entsprechenden Gruppen bzw. Verbindungen, sofern nicht ausdrücklich anders definiert, 1 bis 7, bevorzugt 1 bis 4, Kohlenstoffatome enthalten.

Niederalkanoyloxymethoxycarbonyl ist z.B. Acetoxymethoxycarbonyl oder Pivaloyloxymethoxycarbonyl.

$\alpha$-Aminoniederalkanoyloxymethoxycarbonyl ist z.B. Glycyloxymethoxycarbonyl, Valyloxymethoxycarbonyl oder Leucyloxymethoxycarbonyl.

1-Niederalkoxycarbonyloxyniederalkoxycarbonyl ist z.B. Aethoxycarbonyloxymethoxycarbonyl oder 1-Aethoxycarbonyloxyäthoxycarbonyl.

1-Niederalkoxyniederalkoxycarbonyl ist z.B. Methoxymethoxycarbonyl oder 1-Methoxyäthoxycarbonyl.

Bei einer 2-Oxo-1,3-dioxolen-4-ylmethoxy-Gruppe, welche in 5-Stellung des Dioxolenringes gegebenenfalls durch Niederalkyl oder Phenyl substituiert ist, handelt es sich vor allem um eine 5-Phenyl- und in erster Linie um eine 5-Methyl-2-oxo-1,3-dioxolen-4-ylmethoxy-Gruppe.

Niederalkoxy ist z.B. Methoxy, ferner Aethoxy, n-Propyloxy, Isopropyloxy, n-Butyloxy, Isobutyloxy oder tert.-Butyloxy, sowie n-Pentyloxy, n-Hexyloxy oder n-Heptyloxy, während Aminoniederalkoxy insbesondere 2- oder 3-Aminoniederalkoxy, wie 2-Aminoäthoxy oder 2-Aminopropyloxy, ist.

Niederalkanoyloxy ist z.B. Acetyloxy oder Propionyloxy.

Halogen ist z.B. Fluor, Chlor, Brom oder Jod.

Niederalkylthio ist z.B. Methylthio, Aethylthio oder n-Propylthio, während Aminoniederalkylthio insbesondere 2- oder 3-Aminoniederalkylthio, z.B. 2-Aminoäthylthio, ist.

Niederalkyl ist z.B. Methyl, Aethyl, n-Propyl, Isopropyl, n-Butyl, Isobutyl, sek.-Butyl oder tert.-Butyl, ferner n-Pentyl, n-Hexyl oder n-Heptyl.

Hydroxyniederalkyl ist z.B. Hydroxymethyl, 2-Hydroxyäthyl oder 2,3-Dihydroxypropyl.

Niederalkanoyloxyniederalkyl ist z.B. Acetoxymethyl oder 2-Acetoxyäthyl.

Carboxyniederalkyl ist z.B. Carboxymethyl, 1-Carboxy-, 2-Carboxy- oder 1,2-Dicarboxyäthyl.

Niederalkoxycarbonylniederalkyl ist z.B. entsprechendes Methyl oder Aethyl, wie Methoxycarbonyl- oder Aethoxycarbonylmethyl oder 2-Methoxycarbonyläthyl.

Durch Cyano oder Amino substituiertes Niederalkoxycarbonylniederalkyl ist z.B. entsprechend in 2- oder J-Stellung substituiertes Niederalkoxycarbonylniederalkyl, z.B. 2-Aminoäthoxycarbonylrethyl oder -äthyl sowie 2-Cyanoäthoxycarbonylmethyl oder -äthyl.

Carbamoylniederalkyl ist z.B. Carbamoylmethyl oder 2-Carbamoyläthyl, während Carbamoyloxyniederalkyl z.B. Carbamoyloxymethyl oder 2-Carbamoyloxyäthyl ist.

Cyanoniederalkyl ist z.B. Cyanomethyl, während Halogenniederalkyl z.B. Chlormethyl, Brommethyl, 2-Chloräthyl oder 2,2-Dichloräthyl ist.

Aminoniederalkyl ist z.B. Aminomethyl oder 2-Aminoäthyl, während Niederalkylaminoniederalkyl z.B. Hethylaminomethyl, Aethylaminomethyl, 2-Methylaminoäthyl oder 2-Aethylaminoäthyl und Diniederalkylaminoniederalkyl z.B. Dimethylaminomethyl, 2-Dimethylaminoäthyl oder 2-Diäthylaminoäthyl ist.

Niederalkanoylaminoniederalkyl ist z.B. Acetaminomethyl, 2-Acetaminoäthyl oder Formylaminomethyl.

Amino-carboxy-niederalkyl ist z.B. 2-Amino-2-carboxy-äthyl oder auch 1-Amino-1-carboxymethyl.

Sulfoniederalkyl ist z.B. Sulfomethyl oder 2-Sulfoäthyl.

Niederalkylamino ist z.B. Methylamino, Aethylamino, n-Propylamino, Isopropylamino oder n-Butylamino, während Diniederalkylamino z.B. Dimethylamino, Diäthylamino, Di-n-propylamino oder Diisopropylamino bedeutet.

Niederalkanoylamino ist z.B. Formylamino, Acetylamino oder Propionylamino.

Niederalkoxycarbonyl ist z.B. Methoxycarbonyl oder Aethoxycarbonyl.

Durch Cyano oder Amino substituiertes Niederalkoxycarbonyl ist vor allem entsprechendes in 2- oder 3-Stellung substituiertes Niederalkoxycarbonyl, z.B. 2-Cyanoäthoxycarbonyl oder 2-Aminoäthoxycarbonyl.

Bevorzugt als Rest $R_1$ ist 1-Hydroxyäthyl.

Bevorzugt als Rest $R_2$ sind Carboxyl und unter physiologischen Bedingungen spaltbares verestertes Carboxyl, z.B. Niederalkanoyloxymethoxycarbonyl, wie Acetoxymethoxycarbonyl oder Pivaloyloxymethoxycarbonyl, und 1-Niederalkoxycarbonyloxy-niederalkoxycarbonyl, wie 1-Aethoxycarbonyloxyäthoxycarbonyl.

Bevorzugte Reste $R_3$ sind 3-Pyridyl und durch Hydroxy, Niederalkoxy, Aminoniederalkoxy, Halogen, Niederalkyl, Hydroxyniederalkyl, Carbamoyloxyniederalkyl, Carboxyniederalkyl, Niederalkoxycarbonylniederalkyl, Carbamoylniederalkyl, Aminoniederalkyl, Cyanoniederalkyl, Amino, Carboxy, Niederalkoxycarbonyl oder Carbamoyl substituiertes 3-Pyridyl, z.B. 6-Hydroxypyrid-3-yl, 6-(2-Aminoäthoxy)-pyrid-3-yl, 2-, 4- oder 6-Chlor-pyrid-3-yl, 2-, 4- oder 6-Methyl-pyrid-3-yl, 1-Carboxymethyl-, 1-Carbamoylmethyl- oder 1-Methyl-3-pyridinio, 6-Hydroxymethyl-pyrid-3-yl, 6-Carbamoyloxymethyl-pyrid-3-yl, 6-Aminomethyl-pyrid-3-yl, 2- oder 6-Amino-pyrid-3-yl, 2-, 4-oder 6-Carboxy-pyrid-3-yl, 2-, 4- oder 6-Methoxycarbonyl-pyrid-3-yl, oder 2-, 4- oder 6-Carbamoyl-pyrid-3-yl.

Während der Herstellung sind die in den Verbindungen der Formel I vorhandenen funktionellen Gruppen, wie Hydroxy-, Carboxy- oder Aminogruppen, insbesondere die Hydroxygrupe im Rest $R_1$ und die Carboxylgruppe $R_2$, gegebenenfalls durch Schutzgruppen geschützt, die in der Penem-, Penicillin-, Cephalosporin- und Peptidchemie verwendet werden. Solche Schutzgruppen schützen die betreffenden funktionellen Gruppen vor unerwünschten Kondensationsreaktionen, Substitutionsreaktionen und dergleichen während der Synthese der Verbindung der Formel I aus ihren Vorstufen.

Solche Schutzgruppen sind leicht, d.h. ohne dass unerwünschte Nebenreaktionen stattfinden, beispielsweise solvolytisch oder reduktiv, abspaltbar.

Schutzgruppen dieser Art sowie ihre Einführung und Abspaltung sind beispielsweise beschrieben in

J.F.W. McOmie, "Protective Groups in Organic Chemistry", Plenum Press, London, New York, 1973,
T.W. Greene, "Protective Groups in Organic Synthesis", Wiley, New York, 1981,
"The Peptides", Vol. I, Schroeder und Luebke, Academic Press, London, New York, 1965 und
Houben-Weyl, "Methoden der Organischen Chemie", Band 15/1, Georg Thieme Verlag, Stuttgart, 1974.

So kann eine Hydroxygruppe im Rest $R_1$, ferner eine im Rest $R_3$ vorhandene Hydroxygruppe, beispielsweise durch Acylreste geschützt sein. Geeignete Acylreste sind z.B. gegebenenfalls durch Halogen substituiertes Niederalkanoyl, z.B. Acetyl, Dichloracetyl oder Trifluoracetyl, gegebenenfalls durch Nitro substituiertes Benzoyl, z.B. Benzoyl, 4-Nitrobenzoyl oder 2,4-Dinitrobenzoyl, gegebenenfalls durch Halogen substituiertes Niederalkoxycarbonyl, z.B. 2-Bromäthoxycarbonyl oder 2,2,2-Trichloräthoxycarbonyl, Niederalkenyloxycarbonyl, z.B. Allyloxycarbonyl, Niederalkenyloxyoxalyl, z.B. Allyloxyoxalyl, oder gegebenenfälls durch Nitro substituiertes Phenylniederalkoxycarbonyl, z.B. Benzyloxycarbonyl oder 4-Nitrobenzyloxycarbonyl. Weitere geeignete Hydroxyschutzgruppen sind z.B. trisubstituiertes Silyl, wie Triniederalkylsilyl, z.B. Trimethylsilyl, Dimethyl-(2,3-dimethylbut-2-yl)-silyl oder tert.-Butyl-dimethylsilyl, und 2-Oxa- oder 2-Thiacycloalkyl mit 5 bis 7 Kohlenstoffatomen, z.B. 2-Tetrahydropyranyl oder 2-Tetrahydrofuranyl. Bevorzugt als Hydroxyschutrgruppen sind Triniederalkylsilyl, Niederalkenyloxyoxalyl und Niederalkenyloxycarbonyl.

Eine Carboxylgruppe $R_2$, ferner auch eine im Rest $R_3$ vorhandene Carboxylgruppe, ist üblicherweise in veresterter Form geschützt, wobei die Estergruppe unter schonenden Bedingungen, z.B. unter schonend reduktiven, wie hydrogenolytischen, oder schonend solvolytischen, wie acidolytischen oder insbesondere basisch oder neutral hydrolytischen Bedingungen, leicht spaltbar ist. Solche veresterten Carboxylgruppen enthalten als veresternde Gruppen in erster Linie in 1-Stellung verzweigte oder in 1- oder 2-Stellung geeignet substituierte Niederalkylgruppen. Geeignete In veresterter Form vorliegende Carboxylgruppen sind unter anderen gegebenenfalls durch Nitro oder Niederalkoxy, wie Methoxy, substituiertes Benzyloxycarbonyl, z.B. 4-Nitrobenzyloxycarbonyl oder 4-Methoxybenzyloxycarbonyl, Niederalkanoylmethoxycarbonyl, wie Acetonyloxycarbonyl, Benzoylmethoxycarbonyl, worin die Benzoylgruppe gegebenenfalls, z.B. durch Halogen, wie Brom, substituiert ist, z.B. Phenacyloxycarbonyl, Halogenniederalkoxycarbonyl, wie 2-Halogenniederalkoxycarbonyl, z.B. 2,2,2-Trichloräthoxycarbonyl oder 2-Bromäthoxycarbonyl, Niederalkenyloxycarbonyl, z.B. Allyloxycarbonyl, oder in 2-Stellung durch Niederalkylsulfonyl, Cyano oder trisubstituiertes Silyl, wie Triniederalkylsilyl oder Triphenylsilyl, substituiertes Aethoxycarbonyl, z.B. 2-Methylsulfonyläthoxycarbonyl, 2-Cyanoäthoxycarbonyl, 2-Trimethylsilyläthoxycarbonyl oder 2-(Di-n-butyl-methylsilyl)-äthoxycarbonyl. Bevorzugte geschützte Carboxylgruppen sind die 4-Nitrobenzyloxycarbonyl-, Niederalkenyloxycarbonyl-, insbesondere Allyloxycarbonyl-, und die in 2-Stellung durch Triniederalkylsilyl, z.B. Trimethylsilyl oder Di-n-butyl-methylsilyl, substituierte Aethoxycarbonylgruppe.

Eine geschützte Aminogruppe im Rest $R_3$ kann beispielsweise in Form einer leicht spaltbaren Acylaminogruppe oder als Azidogruppe vorliegen. In einer entsprechenden Acylaminogruppe ist Acyl beispielsweise der Acylrest einer organischen Säure mit z.B. bis zu 12 Kohlenstoffatomen, insbesondere einer gegebenenfalls z.B. durch Halogen oder Phenyl, substituierten Niederalkancarbonsäure oder insbesondere eines Kohlensäurehalbesters. Solche Acylgruppen sind beispielsweise Halogenniederalkanoyl, wie 2-Halogenacetyl, insbesondere 2,2,2-Trifluor- oder 2,2,2-Trichloracetyl, Niederalkenyloxycarbonyl, z.B. Allyloxycarbonyl, gegebenenfalls in 1- oder 2-Stellung substituiertes Niederalkoxycarbonyl, wie Niederalkoxycarbonyl, z.B. tert.Butoxycarbonyl, gegebenenfalls substituiertes Benzyloxycarbonyl, z.B. 4-Nitrobenzyloxycarbonyl, 2-Halogenniederalkoxycarbonyl, z.B. 2,2,2-Trichloräthoxycarbonyl oder 2-Bromäthoxycarbonyl, oder 2-(trisubstituiertes Silyl)-äthoxycarbonyl, wie 2-Triniederalkylsilyläthoxycarbonyl, z.B. 2-Trimethylsilyläthoxycarbonyl oder 2-(Di-n-butylmethylsilyl)-äthoxycarbonyl. Bevorzugte geschützte Aminogruppen sind Azido, Niederalkenyloxycarbonylamino, z.B. Allyloxycarbonylamino, und gegebenenfalls durch Nitro substituiertes Benzyloxycarbonylamino.

Eine monosubstituierte Aminogruppe, z.B. Niederalkylamino, ist in gleicher Weise wie eine Aminogruppe, beispielsweise als substituiertes Niederalkenyloxycarbonylamino, geschützt.

Eine geschützte Sulfogruppe im Rest $R_3$ ist in gleicher Weise wie eine geschützte Carboxylgruppe, insbesondere in veresterter Form geschützt.

Salze von erfindungsgemässen Verbindungen sind in erster Linie pharmazeutisch annehmbare, nicht-toxische Salze von Verbindungen der Formel I. Solche Salze werden beispielsweise von den in Verbindungen der Formel I vorhandenen sauren Gruppen, z.B. Carboxylgruppen, gebildet und sind in erster Linie Metall- oder Ammoniumsalze, wie Alkalimetall- und Erdalkalimetall-, z.B. Natrium-, Kalium-, Magnesium- oder Kalziumsalze, sowie Ammoniumsalze mit Ammoniak oder geeigneten organischen Aminen, wie Niederalkylaminen, z.B. Triäthylamin, Hydroxyniederalkylaminen, z.B. 2-Hydroxyäthylamin, Bis-(2-hydroxyäthyl)-amin, Tris-(2-hydroxyäthyl)-amin oder 2-Amino-1,3-dihydroxy-2-hydroxymethyl-propan ("Tris"), basischen aliphatischen Estern von Carbonsäuren, z.B. 4-Aminobenzoesäure-2-diäthylaminoäthylester, Niederalkylenaminen, z.B. 1-Aethyl-piperidin, Cycloalkylaminen, z.B. Dicyclohexylamin, oder Benzylaminen, z.B. N,N'-Dibenzyläthylendiamin, Dibenzylamin oder N-Benzyl-β-phenäthylamin. Verbindungen der Formel I mit einer basischen Gruppe,

z.B. mit einer Aminogruppe, können Säureadditionssalze, z.B. mit anorganischen Säuren, wie Salzsäure, Schwefelsäure oder Phosphorsäure, oder mit geeigneten organischen Carbon- oder Sulfonsäure, z.B. Essigsäure, Bernsteinsäure, Fumarsäure, Maleinsäure, Weinsäure, Oxalsäure, Zitronensäure, Benzoesäure, Mandelsäure, Aepfelsäure, Ascorbinsäure, Methansulfonsäure oder 4-Toluolsulfonsäure bilden. Verbindungen der Formel I mit einer sauren und mit einer basischen Gruppe können auch in Form von inneren Salze, d.h. in zwitterionischer Form, vorliegen. Beispielsweise können Verbindungen der Formel I mit einer Pyridinio-Gruppe $R_3$ als Carboxylat ($R_2$ ist $COO^{\ominus}$) vorliegen.

Zur Isolierung oder Reinigung können auch pharmazeutisch ungeeignete Salze Verwendung finden. Zur therapeutischen Anwendung gelangen nur die pharmazeutisch annehmbaren, nicht-toxischen Salze, die deshalb bevorzugt sind.

Die Penem-Verbindungen der Formel I können im Rest $R_1$ ein zusätzliches Chiralitätszentrum besitzen. Beispielsweise kann 1-Hydroxyäthyl als Substituent $R_1$ in der R-, in der S- oder in der racemischen R,S-Konfiguration vorliegen. In bevorzugten Penem-Verbindungen der Formel I weist 1-Hydroxyäthyl die R-Konfiguration auf. Die Erfindung betrifft demgemäss die reinen Diastereomeren und Mischungen der Diastereomeren von Verbindungen der Formel I, welche im Rest $R_1$ ein zusätzliches Chiralitätszentrum aufweisen.

Die Verbindungen der Formel I weisen wertvolle pharmakologische Eigenschaften auf oder können als Zwischenprodukte zur Herstellung von solchen Verbindungen mit wertvollen pharmakologischen Eigenschaften verwendet werden. Verbindungen der Formel I, und pharmakologisch annehmbare Salze von solchen Verbindungen mit salzbildenden Gruppen haben antibakterielle Wirkungen. Beispielsweise sind sie in vitro gegen grampositive und gramnegative Kokken, z.B. Staphylokokken (inkl. Methicillin-resistente Staphylokokken), wie Staphylococcus aureus, Streptococcus pyogenes, Streptococcus pneumoniae, Streptococcus faecalis und Neisseria spec., in minimalen Konzentrationen von <0,01 bis ca. 8 µg/ml und gegen gramnegative Stäbchenbakterien, wie Enterobacteriaceae und Haemophilus influenzae, Pseudomonas und Anaerobier, z.B. Bacteroides sp., in minimalen Konzentrationen von ca. 0,02 bis ca. 64 µg/ml wirksam In vivo, bei der systemischen Infektion der Maus, z.B. durch Staphylococcus aureus, ergeben sich bei parenteraler oder oraler Applikation erfindungsgemässer Verbindungen $ED_{50}$-Werte von ca 0,5 bis ca. 15 mg/kg.

Die neuen Verbindungen können als oral oder parenteral applizierbare antibakterielle Breitspektrum-Antibiotika, z.B. in Form von entsprechenden pharmazeutischen Präparaten, zur Behandlung von Infektionen Verwendung finden.

Die Erfindung betrifft hauptsächlich Verbindungen der Formel I, worin $R_1$ Hydroxymethyl oder 1-Hydroxyäthyl ist, $R_2$ Carboxyl oder unter physiologischen Bedingungen spaltbares verestertes Carboxyl bedeutet und $R_3$ 3-Pyridyl, durch Hydroxy, Niederalkoxy, Aminoniederalkoxy, Halogen, Niederalkyl, Hydroxyniederalkyl, Carboxyniederalkyl, Niederalkoxycarbonylniederalkyl, Carbamoyloxyniederalkyl, Carbamoylniederalkyl, Aminoniederalkyl, Cyanoniederalkyl, Amino, Carboxy, Niederalkoxycarbonyl oder Carbamoyl substituiertes 3-Pyridyl bedeutet, und Salze von Verbindungen der Formel I.

Die Erfindung betrifft in erster Linie Verbindungen der Formel I, worin $R_1$ Hydroxymethyl oder 1-Hydroxyäthyl ist, $R_2$ Carboxyl oder unter physiologischen Bedingungen spaltbares verestertes Carboxyl bedeutet und $R_3$ 3-Pyridyl oder durch Niederalkyl oder Halogen substituiertes 3-Pyridyl darstellt, und Salze von Verbindungen der Formel I.

Die Erfindung betrifft in erster Linie die in den Beispielen genannten Verbindungen der Formel I und deren Salze, insbesondere deren pharmazeutisch annehmbare Salze.

Die Verbindungen der vorliegenden Erfindung können nach an sich bekannten Verfahren hergestellt werden.

Die neuen Verbindungen werden z.B. hergestellt, indem man eine Ylid-Verbindung der Formel

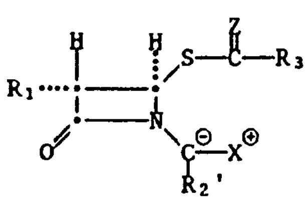

(II),

worin $R_1$ und $R_3$ die unter Formel I angegebenen Bedeutungen haben, $R_2'$ eine geschützte Carboxylgruppe darstellt, Z Sauerstoff oder Schwefel bedeutet und $X^{\oplus}$ entweder eine dreifach substituierte Phosphoniogruppe oder eine zweifach veresterte Phosphonogruppe zusammen mit einem Kation darstellt, ringschliesst, oder eine Verbindung der Formel

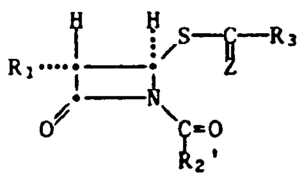

(III),

worin $R_1$ und $R_3$ die unter Formel I und $R_2'$ und Z die unter Formel II angegebenen Bedeutungen haben, mit einer organischen Verbindung des dreiwertigen Phosphors behandelt, und, wenn erwünscht oder notwendig, in einer erhältlichen Verbindung der Formel I geschützte funktionelle Gruppen in die freien funktionellen Gruppen überführt, und/oder, wenn erwünscht, in einer erhältlichen Verbindung der Formel I eine freie Carboxylgruppe $R_2$ in eine unter physiologischen Bedingungen spaltbare veresterte Carboxylgruppe $R_2$ überführt, und/oder, wenn erwünscht, in einer erhältlichen Verbindung der Formel I einen Rest $R_3$ in einen anderen Rest $R_3$ überführt, und/oder, wenn erwünscht, eine erhältliche Verbindung mit salzbildender Gruppe in ein Salz oder ein erhältliches Salz in die freie Verbindung oder in ein anderes Salz überführt.

In den Ausgangsverbindungen der Formeln II und III sind funktionelle Gruppen, wie eine freie Hydroxygruppe im Rest $R_1$ sowie weitere im Rest $R_3$ enthaltene funktionelle Gruppen, vorzugsweise durch konventionelle Schutzgruppen, z.B. durch die oben genannten, geschützt.

Cyclisierung der Verbindung der Formel II

Die Gruppe $X^{\oplus}$ im Ausgangsmateriel der Formel II ist eine der bei Wittig-Kondensationsreaktionen gebräuchlichen Phosphonio- oder Phosphonogruppen, insbesondere eine Triaryl-, z.B. Triphenyl-, oder Triniederalkyl-, z.B. Tri-n-butylphosphoniogruppe, oder eine durch Niederalkyl, z.B. Aethyl, zweifach veresterte Phosphonogruppe, wobei das Symbol $X^{\oplus}$ für den Fall der Phosphonogruppe zusätzlich das Kation einer starken Base, insbesondere ein geeignetes Metall-, wie Alkalimetallion, z.B. das Lithium-, Natrium- oder Kaliumion, umfasst. Bevorzugt als Gruppe $X^{\oplus}$ sind einerseits Triphenylphosphonio und andererseits Diäthylphosphono zusammen mit einem Alkalimetall-, z.B. dem Natriumion.

Der Ringschluss kann spontan, d.h. bei der Herstellung der Ausgangsstoffe, oder durch Erwärmen, z.B. in einem Temperaturbereich von etwa 30°C bis 160°C, vorzugsweise von etwa 80°C bis etwa 120°C, erfolgen. Die Reaktion wird vorzugsweise in einem geeigneten inerten Lösungsmittel, wie in einem aliphatischen, cycloaliphatischen oder aromatischen Kohlenwasserstoff, z.B. Cyclohexan, Benzol oder Toluol, einem halogenierten Kohlenwasserstoff, z.B. Methylenchlorid, einem Aether, z.B. Diäthyläther, einem cyclischen Aether, z.B. Dioxan oder Tetrahydrofuran, einem Carbonsäureamid, einem Diniederalkylsulfoxid, oder einem Niederalkanol oder in einem Gemisch davon, und, falls notwendig, in einer Inertgas-, z.B. Stickstoffatmosphäre, durchgeführt.

Cyclisierung der Verbindung der Formel III

Eine organische Verbindung des dreiwertigen Phosphors leitet sich z.B. von phosphoriger Säure ab und ist insbesondere ein Ester derselben mit einem Niederalkanol, z.B. Methanol oder Aethanol, und/oder einer gegebenenfalls substituierten aromatischen Hydroxyverbindung, z.B. Phenol oder Brenzcatechin, oder ein Amidester derselben der Formel $P(OR_a)_2N(R_b)_2$, worin $R_a$ und $R_b$ unabhängig voneinander Niederalkyl, z.B. Methyl, oder Aryl, z.B. Phenyl, bedeuten. Bevorzugte Verbindungen des dreiwertigen Phosphors sind Triniederalkylphosphite, z.B. Trimethylphosphit oder Triäthylphosphit.

Die Reaktion wird vorzugsweise in einem inerten Lösungsmittel, wie einem aromatischen Kohlenwasserstoff, z.B. Benzol oder Toluol, einem Aether, z.B. Dioxan oder Tetrahydrofuran, oder einem halogenierten Kohlenwasserstoff, z.B. Methylenchlorid oder Chloroform, bei einer Temperatur von etwa 20° bis 140°C, bevorzugt von etwa 80° bis etwa 120°C, durchgeführt, wobei man 1 Moläquivalent einer Verbindung der Formel III mit mindestens 2 Moläquivalenten der Phosphorverbindung umsetzt. Beispielsweise legt man die Verbindung der Formel III in einem inerten Lösungsmittel vor und tropft die Phosphorverbindung, vorzugsweise in dem gleichen inerten Lösungsmittel gelöst, über einen längeren Zeitraum, z.B. während eines Zeitraums von 2 bis 4 Stunden, hinzu.

In einer bevorzugten Ausführungsform des Verfahrens stellt man das Ausgangsmaterial der Formel III wie weiter unten angegeben her und setzt es ohne Isolierung aus dem Reaktionsgemisch mit der organischen Verbindung des dreiwertigen Phosphors um, wobei die Endprodukte der Formel I entstehen.

Verfahrensgemäss erhältliche Verbindungen der Formel I können in an sich bekannter Weise in andere Verbindungen der Formel I überführt werden.

So können in einer erhältlichen Verbindung der Formel I, worin eine oder mehrere funktionelle Gruppen

geschützt sind, diese, z.B. geschützte Carboxyl-, Hydroxy- und/oder Aminogruppen, in an sich bekannter Weise mittels Solvolyse, insbesondere Hydrolyse, Alkoholyse oder Acidolyse, oder mittels Reduktion, insbesondere Hydrogenolyse oder chemische Reduktion, gegebenenfalls stufenweise oder gleichzeitig freigesetzt werden.

In einer verfahrensgemäss erhältlichen Verbindung der Formel I, worin $R_2$ eine geschützte Carboxylgruppe bedeutet und/oder worin der Rest $R_3$ geschütztes Carboxyl als Substituenten enthält, kann die geschützte Carboxylgruppe in an sich bekannter Weise freigesetzt werden. So kann man in 2-Stellung durch eine trisubstituierte Silylgruppe substituiertes Aethoxycarbonyl z.B. durch Behandeln mit einer Carbonsäure, wie Ameisensäure oder Trifluoressigsäure, gegebenenfalls unter Zugabe einer nucleophilen Verbindung, wie Phenol oder Anisol, in freies Carboxyl überführen. Gegebenenfalls substituiertes Benzyloxycarbonyl kann z.B. mittels Hydrogenolyse, d.h. durch Behandeln mit Wasserstoff in Gegenwart eines metallischen Hydrierkatalysators, wie eines Palladiumkatalysators, gespalten werden. Ferner kann man geeignet substituiertes Benzyloxycarbonyl, wie 4-Nitrobenzyloxycarbonyl, auch mittels chemischer Reduktion, z.B. durch Behandeln mit einem Alkalimetall-, z.B. Natriumdithionit, oder mit einem reduzierenden Metall, z.B. Zinn, üblicherweise in Gegenwart eines Wasserstoff-abgebenden Mittels, das zusammen mit dem Metall nascierenden Wasserstoff zu erzeugen vermag, wie einer geeigneten Carbonsäure, z.B. einer gegebenenfalls, z.B. durch Hydroxy, substituierten Niederalkancarbonsäure, z.B. Essigsäure, oder eines Alkohols oder Thiols, wobei man vorzugsweise Wasser zugibt, in freies Carboxyl überführen. Die Abspaltung einer Allylschutzgruppe kann z.B. durch Umsetzung mit einer Verbindung des Palladiums, z.B. Tetrakis-(triphenylphosphin)-palladium, gegebenenfalls in Gegenwart von Triphenylphosphin und unter Zusatz eines Allylgruppenakzeptors, wie einer Carbonsäure, z.B. 2-Aethylhexansäure, oder eines Salzes davon oder Tributylzinnhydrid oder Dimedon, erfolgen. Durch Behandeln mit einem reduzierenden Metall oder Metallsalz, wie oben beschrieben, kann man auch 2-Halogenniederalkoxycarbonyl (gegebenenfalls nach Umwandlung einer 2-Bromniederalkoxycarbonylgruppe in eine entsprechende 2-Jodniederalkoxycarbonylgruppe) oder Benzoylmethoxycarbonyl in freies Carboxyl umwandeln, wobei Benzoylmethoxycarbonyl auch durch Behandeln mit einem nucleophilen, vorzugsweise salzbildenden Reagenz, wie Natriumthiophenolat oder Natriumjodid, gespalten werden kann. Substituiertes 2-Silyläthoxycarbonyl kann auch durch Behandeln mit einem das Fluoridanion liefernden Salz der Fluorwasserstoffsäure, wie einem Alkalimetallfluorid, z.B. Natriumfluorid, in Anwesenheit eines makrocyclischen Polyäthers ("Kronenäther") oder mit einem Fluorid einer organischen quaternären Base, wie Tetraniederalkylammoniumfluorid, z.B. Tetrabutylammoniumfluorid, in freies Carboxyl überführt werden. Eine in 2-Stellung durch Niederalkylsulfonyl oder Cyano substituierte Niederalkoxycarbonylgruppe kann z.B. durch Behandeln mit einem basischen Mittel, wie einem Alkalimetall- oder Erdalkalimetallhydroxid oder -carbonat, z.B. Natrium-oder Kaliumhydroxid oder Natrium- oder Kaliumcarbonat, in freies Carboxyl übergeführt werden.

Andererseits können auch Verbindungen der Formel I, worin $R_2$ Carboxyl bedeutet, in Verbindungen der Formel I überführt werden, worin $R_2$ eine unter physiologischen Bedingungen spaltbare veresterte Carboxylgruppe darstellt. Solche Ester können z.B. durch Umsetzung eines gegebenenfalls in situ hergestellten Salzes der Säure mit einem reaktionsfähigen Ester eines entsprechenden Alkohols und einer starken anorganischen Säure, wie Schwefelsäure, oder einer starken organischen Sulfonsäure, wie 4-Toluolsulfonsäure, hergestellt werden. Ferner kann man in Verbindungen der Formel I, die eine in veresterter Form geschützte Carboxylgruppe enthalten, die Carboxylschutzgruppe wie oben beschrieben abspalten und eine entstandene Verbindung der Formel I mit einer freien Carboxylgruppe oder ein Salz davon durch Umsetzen mit dem reaktionsfähige Ester eines entsprechenden Alkohols in eine Verbindung der Formel I überführen, worin $R_2$ eine unter physiologischen Bedingungen spaltbare veresterte Carboxylgruppe darstellt.

In verfahrensgemäss erhältlichen Verbindungen der Formel I, worin der Rest $R_1$ und/oder gegebenenfalls der Rest $R_3$ geschütztes Hydroxyl als Substituenten enthält, kann die geschützte Hydroxygruppe in an sich bekannter Weise in die freie Hydroxygruppe überführt werden. Beispielsweise wird eine durch eine geeignete Acylgruppe oder eine organische Silylgruppe geschützte Hydroxygruppe wie eine entsprechend geschützte Aminogruppe freigesetzt (s.u.); eine Triniederalkylsilylgruppe kann z.B. auch mit Tetrabutylammoniumfluorid und Essigsäure abgespalten werden (unter diesen Bedingungen werden durch trisubstituiertes Silyläthoxy geschützte Carboxygrupen nicht gespalten).

In einer erfindungsgemäss erhältlichen Verbindung der Formel I mit einer geschützten Aminogruppe im Rest $R_3$ kann diese in an sich bekannter Weise, z.B. je nach Art der Schutzgruppe, vorzugsweise mittels Solvolyse oder Reduktion, in die freie Aminogruppe übergeführt werden. Beispielsweise kann 2-Halogenniederalkoxycarbonylamino (gegebenenfalls nach Umwandlung einer 2-Bromniederslkoxycarbonylaminogruppe in eine 2-Jodniederalkoxycarbonylominogruppe) und 4-Nitrobenzyloxycarbonylamino durch Behandeln mit einem geeigneten chemischen Reduktionsmittel, wie Zink in Gegenwort einer geeigneten Carbonsäure, wie wässriger Essigsäure, oder katalytisch mit Wasserstoff in Gegenwart eines Palladiumkatalysators oder durch Behandeln mit einem Alkalimetall-, z.B. Natriumdithionit gespalten werden. Gegebenenfalls substituiertes Benzyloxycarbonylomino kann z.B. mittels Hydrogenolyse, d.h. durch Behandeln mit Wasserstoff in Gegenwart eines geeigneten Hydrierkatalysators, wie eines Palladiumkatalysators, und Allyloxycarbonylamino durch Umsetzen mit einer verbindung des Palladiums, z.B. Tetrakis-(triphenylphosphin)-palladium, gegebenenfalls in Gegenwart von Triphenylphosphin und in Gegenwart eines Allylgruppenakzeptors, wie einer Carbonsäure, z.B. 2-Aethylhexansäure, oder eines Salzes davon oder Tributylzinnhydrid oder Dimedon, gespalten werden. Eine durch 2-Halogenniederalkanoyl, z.B. 2-Chloracetyl. geschützte Aminogruppe kann durch Behandeln mit Thioharnstoff in Gegenwart einer Base oder mit einem Salz, wie einem Alkalimetall-Salz. des Thioharnstoffs und anschliessender Solvolyse, wie Alkoholyse oder Hydrolyse, des entstandenen Kondensationsproduktes freigesetzt werden. Eine durch 2-substituiertes Silyläthoxycarbonyl geschützte Aminogruppe kann durch Behandeln mit einem Fluoridanionen liefernden Salz der Fluorwasserstoffsäure, wie einem Alkalimetallfluorid, z.B. Natriumfluorid, in Anwesenheit eines makrocyclischen Polyäthers ("Kronenäther") oder mit einem Fluorid einer organischen quaternären Base, wie Tetraniederalkylammoniumfluorid, z.B. Tetraäthylammoniumfluorid, in die freie Aminogruppe übergeführt werden. Eine in Form einer Azidogruppe geschützte Aminogruppe wird z.B. durch Reduktion in freies Amino überführt, beispielsweise durch katalytische Hydrierung mit Wasserstoff in Gegenwart eines Hydrierkatalysators, wie Platinoxid oder Palladium, oder durch Behandeln mit Zink in Gegenwart einer Säure, wie Essigsäure.

In Verbindungen der Formel I kann man weiterhin einen Rest $R_3$ in einen anderen Rest $R_3$ überführen.

So kann beispielsweise in Verbindungen der Formel I, worin der Rest $R_3$ durch eine Carboxylgruppe substituiert ist, diese Carboxylgruppe nach an sich bekannten Verfahren in eine funktionell abgewandelte Carboxylgruppe, wie in eine veresterte Carboxylgruppe oder in Carbamoyl, überführt werden. Beispielsweise erhält man durch Umsetzen einer Verbindung der Formel I, worin der Rest $R_3$ durch Carboxyl substituiert ist, mit einem gegebenenfalls substituierten Niederalkanol eine Verbindung der Formel I, worin $R_3$ durch gegebenenfalls substituiertes Niederalkoxycarbonyl substituiert ist, wobei man vorzugsweise in Gegenwart eines geeigneten Kondensationsmittels, z.B. eines Carbodiimids, arbeitet oder das entstehende Wasser durch azeotrope Destillation entfernt. Andererseits kann man Carboxylgruppen an Resten $R_3$ auch in reaktionsfähige funktionelle Derivate, wie gemischte Anhydride, z.B. Säurehalogenide, oder aktivierte Ester, überführen und diese durch Umsetzen mit einem entsprechenden Alkohol, z.B. einem gegebenenfalls substituierten Niederalkanol, oder mit Ammoniak in entsprechend veresterte Carboxylgruppen oder in eine Carbamoylgruppe umwandeln, wobei man bei Verwendung von gemischten Anhydriden vorzugsweise in Gegenwart eines säurebindenden Mittels, wie eines aromatischen oder tertiären Amins oder eines Alkalimetall- oder Erdalkalimotellcarbonats, arbeitet.

In Verbindungen der Formel I, worin der Rest $R_3$ durch Amino substituiert ist, kann die Aminogruppe in eine substituierte Aminogruppe, z.B. eine Niederalkylamino-, Diniederalkylamino- oder Niederalkanoylaminogruppe, überführt werden. Die Ueberführung in eine Niederalkylamino- oder Diniederalkylominogruppe erfolgt beispielsweise durch Reaktion mit einem reaktionsfähigen veresterten Niederalkanol, beispielsweise einem Niederalkylhalogenid oder -sulfonat, in Gegenwart eines basischen Kondensationsmittels, wie eines Hydroxids oder Carbonats eines Alkali- oder Erdalkalimetalls oder einer heteroaromatischen Stickstoffbase, z.B. Pyridin. In analoger Weise kann Amino durch Behandeln mit dem reaktionsfähigen funktionellen Derivat einer Niederalkancarbonsäure, z.B. dem entsprechenden Carbonsäurehalogenid, in Niederalkanoylamino umgewandelt werden. Verbindungen der Formel I mit einem unsubstituierten Pyridin-Stickstoffatom im Rest $R_3$ können ferner durch besetzen mit den reaktionsfähigen Ester eines gegebenenfalls substituierten Niederalkanols, beispielsweise eines Benzolsulfonats, p-Toluolsulfonats, Methansulfonats oder Halogenids, z.B. Chlorids, davon in Verbindungen der Formel I überführt werden, worin $R_3$ ein am Stickstoffatom durch gegebenenfalls substituiertes Niederalkyl substituierter Pyridyl-Rest ist.

Salze von Verbindungen der Formel I mit salzbildenden Gruppen können in an sich bekannter Weise hergestellt werden. So kann man Salze von Verbindungen der Formel I mit einer freien Carboxyl- oder Sulfogruppe z.B. durch Behandeln mit Metallverbindungen, wie Alkalimetallsalzen von geeigneten organischen Carbonsäure, z.B. dem Natriumsalz der $\alpha$-Aethylcapronsäure, oder mit anorganischen Alkali- oder Erdalkalimetallsalzen, z.B. Natriumhydrogencarbonat, oder mit Ammoniak oder mit einem geeigneten organischen Amin bilden, wobei man vorzugsweise stöchiometrische Mengen oder nur einen kleinen Ueberschuss des salzbildenden Mittels verwendet. Säureadditionssalze von Verbindungen der Formel I erhält man in üblicher Weise, z.B. durch Behandeln mit einer geeigneten Säure oder einem geeigneten Anionenaustauscherreagenz. Innere Salze von Verbindungen der Formel I können z.B. durch Neutralisieren von Salzen, wie Säureadditionssalzen, auf den isoelektrischen Punkt, z.B. mit schwachen Basen, oder durch Behandeln mit Ionenaustauschern gebildet werden.

Salze können in üblicher Weise in die freien Verbindungen überführt werden, Metall- und Ammoniumsalze z.B. durch Behandeln mit geeigneten Säuren und Säureadditionssalze z.B. durch Behandeln mit einem geeigneten basischen Mittel.

Erhaltene Gemische von isomeren Verbindungen können in an sich bekannter Weise in die einzelnen Isomere aufgetrennt werden. Gemische diastereomerer Verbindungen lassen sich beispielsweise durch fraktionierte Kristallisation trennen. Erhaltene Racemate kann man z.B. mit einem optisch aktiven Hilfsstoff reagieren lassen, das entstandene Gemisch zweier diastereomerer Verbindungen durch fraktionierte Kristallisation trennen und die einzelnen Diastereomere in die optisch aktiven Verbindungen spalten.

Bei allen nachträglichen Umwandlungen erhaltener Verbindungen der Formel I werden solche Reaktionen bevorzugt, die unter gemässigt alkalischen oder insbesondere neutralen Bedingungen erfolgen.

Das Verfahren umfasst such diejenigen Ausführungsformen, wonach als Zwischenprodukte anfallende Verbindungen als Ausgangsstoffe verwendet und die restlichen Verfahrensschritte mit diesen durchgeführt werden, oder das Verfahren auf irgendeiner Stufe abgebrochen wird. Ferner können Ausgangsstoffe in Form von Derivaten verwendet oder in situ, gegebenenfalls unter den Reaktionsbedingungen, hergestellt werden.

Die Ausgangsverbindungen der Formeln II und III können, wie in dem folgenden Reaktionsschema I angegeben, hergestellt werden:

Reaktionsschema I

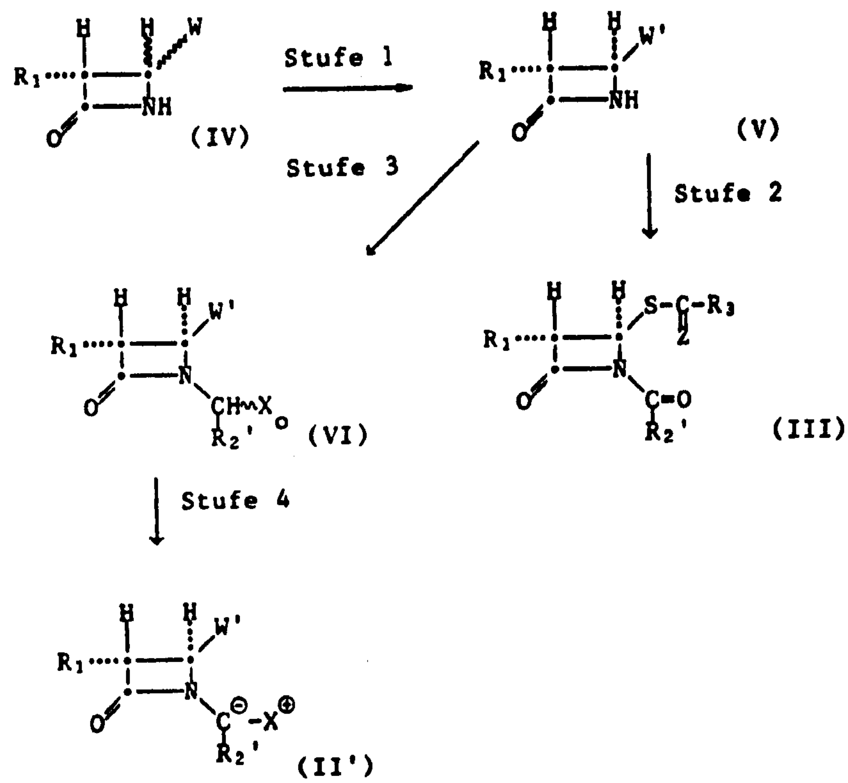


In den Verbindungen der Formel V, VI und II′ steht W′ für den Rest -S-C(=Z)-$R_3$ oder für Triphenylmethylthio oder Niederalkanoylthio.

Stufe 1

Geeignete Ausgangsverbindungen der Formel IV, worin W ein durch nucleophile Reaktion leicht austauschbarer Rest, z.B. Niederalkanoyloxy, wie Acetyloxy, oder Sulfonyloxy $R_o$-$SO_2$-, worin $R_o$ z.B. gegebenenfalls durch Hydroxy substituierten Niederalkyl, wie Methyl, tert.Butyl oder 2-Hydroxyäthyl, ist, sind beispielsweise aus der veröffentlichten Europäischen Patentanmeldung Nr. 82113, der Deutschen Offenlegungsschrift Nr. 3 224 055 und der Deutschen Offenlegungsschrift Nr. 3 013 997 bekannt oder können in dazu analoger Weise hergestellt werden.

Eine den Rest -S-C(=Z)-$R_3$ einführende Verbindung ist beispielsweise eine Säure der Formel $R_3$-C(=Z)-SH oder insbesondere ein Salz, z.B. ein Alkalimetallsalz, wie das Natrium- oder Kaliumsalz, davon. Die Substitution kann in einem organischen Lösungsmittel, wie in einem Niederalkanol, einem Niederalkancarbonsäureamid, einem cyclischen Aether, oder in einem ähnlichen inerten Lösungsmittel bei Raumtemperatur oder bei etwas erhöhter oder erniedrigter Temperatur, z.B. bei etwa 0° bis etwa 40°C, durchgeführt werden. Die Einführung eines Triphenylmethylthio- oder Niederalkanoylthio-Restes W′ erfolgt in analoger Weise durch Umsetzen mit

einem Alkalimetallsalz, z.B. dem Natriumsalz, einer Thioniederalkancarbonsäure, z.B. Thioessigsäure, oder des Triphenylmethylmercaptans.

Stufe 2

Eine Ausgangsverbindung der Formel (III) wird erhalten, indem man ein Azetidinon der Formel (V), in der W′ für den Rest -S-C(=Z)-$R_3$ steht, mit einer Säure der Formel $R_2$′-COOH oder insbesondere einem reaktionsfähigen Derivat, wie einem Ester öder Säurehalogenid, z.B. dem Säurechlorid, davon bei einer Temperatur von -50° bis 80°C, bevorzugt bei -20° bis 0°C, in einem inerten Lösungsmittel, wie einem der bei der Umsetzung von Verbindungen der Formel (III) zu Verbindungen der Formel I genannten, behandelt. Bei Verwendung eines Säurehalogenids arbeitet man vorzugsweise in Gegenwart eines säurebindenden Mittels, wie eines tertiären aliphatischen Amins, eines aromatischen Amins, oder insbesondere eines Alkalimetall- oder Erdalkalimetallcarbonats oder -hydrogencarbonats.

Verbindungen der Formel (V), worin W′ für Triphenylmethylthio oder Niederalkanoylthio steht, können in die Ausgangsverbindungen der Formel V, worin W′ für den Rest -S-C(=Z)-$R_3$ steht, überführt werden, indem man sie in Gegenwart einer Base, z.B. Pyridin oder Tri-n-butylamin, in einem geeigneten Lösungsmittel, z.B. Diäthyläther oder Methanol, mit einem Salz der Formel MA, worin M für ein Uebergangsmetallkation, insbesondere für das Silberkation, steht, und A ein gebräuchliches Anion darstellt, welches die Löslichkeit des Salzes MA in dem gewählten Lösungsmittel begünstigt, z.B. das Nitrat-, Acetat- oder Fluorid-Anion, umsetzt, und das entstandene Salz der Formel

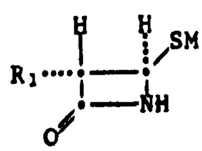

(V′),

mit einem den Rest $R_3$-C(-2)- einführenden Acylierungsmittel, z.B. mit der Säure $R_3$-C(=Z)-OH oder einem reaktionofähigen funktionellen Derivat, wie einem Säurehalogenid, z.B. dem Chlorid oder Bromid, Azid oder Anhydrid davon, behandelt. Die Acylierung erfolgt,wenn ein reaktionsfähiges funktionelles Derivat der Säure der Formel $R_3$-C(=Z)-OH, z.B. das Säurechlorid, eingesetzt wird, in einem inerten Lösungsmittel, wie einem chlorierten Kohlenwasserstoff, oder einem Aether, bei Raumtemperatur oder unter Erhitzen oder Kühlen, z.B. in einem Temperaturbereich von ca. -50° bis ca. +60°C, insbesondere bei ca. -30° bis ca. +20°C.

Stufe 3

Verbindungen der Formel VI, worin $X_o$ für eine reaktionsfähige veresterte Hydroxygruppe, insbesondere für Halogen, z.B. Chlor oder Brom, steht, werden hergestellt, indem man eine Verbindung der Formel V mit einer Glyoxylsäure-Verbindung der Formel $R_2$′-CHO oder einem geeigneten Derivat davon, wie einem Hydrat, Hemihydrat oder Halbacetal, z.B. einem Halbacetal mit einem Niederalkanol, z.B. Methanol oder Aethanol, umsetzt, und in einer erhaltenen Verbindung der Formel VI, worin $X_o$ für Hydroxy steht, die Hydroxygruppe in eine reaktionsfähige veresterte Hydroxygruppe umwandelt. Die Verbindungen der Formel VI werden üblicherweise als Gemisch der beiden Isomere

[bezüglich der Gruppierung -CH($R'_2$)~$X_o$]

erhalten.

Die Anlagerung der Glyoxylsäureester-Verbindung an das Stickstoffatom des Lactarrings in der Verbindung der Formel V findet bei Raumtemperatur oder, falls notwendig, unter Erwärmen, statt. Bei Verwendung des Hydrats der Glyoxylsäure-Verbindung wird Wasser gebildet, das, wenn notwendig, durch Destillation, z.B. azeotrop, oder durch Verwendung eines geeigneten Dehydratationsmittels enfernt wird. Vorzugsweise arbeitet man in Gegenwart eines geeigneten inerten Lösungsmittels oder Lösungsmittelgemisches.

Die Ueberführung einer Hydroxygruppe $X_o$ in eine reaktionsfähige veresterte Hydroxygruppe $X_o$ in einer Verbindung der Formel VI wird durch Behandeln mit einem geeigneten Veresterungsmittel durchgeführt, z.B. mit einem Thionylhalogenid, z.B. -chlorid, vorzugsweise in Gegenwart eines basischen, in erster Linie organischen basischen Mittels, wie eines aliphatischen tertiären Amins, oder einer heterocyclischen Base vom Pyridintyp. Vorzugsweise arbeitet man in Gegenwart eines geeigneten Lösungsmittels, z.B. Dioxan oder Tetrahydrofuran, oder eines Lösungsmittelgemisches, wenn notwendig unter Kühlen, z.B. bei etwa -30° bis

etwa 30°C.

Stufe 4

Das Ausgangsmaterial der Formel (II) wird erhalten, indem man eine Verbindung der Formel (VI) mit einer geeigneten Phosphin-Verbindung, wie einem Triniederalkylphosphin, z.B. Tri-n-butyl-phosphin, oder einem Triaryl-phosphin, z.B. Triphenylphosphin, oder mit einer geeigneten Phosphit-Verbindung, wie einem Triniederalkylphosphit, z.B. Triäthylphosphit, oder einem Alkalimetalldiniederalkylphosphit, z.B. -diäthylphosphit, behandelt, und eine gegebenenfalls erhältlichen Verbindung der Formel (II′), worin W′ für Triphenylmethylthio oder Niederalkanoylthio steht, in eine Verbindung der Formel (II′) überführt, worin W′ für den Rest -S-C(=Z)-$R_3$ steht.

Die Umsetzung mit der Phosphin- bzw. Phosphit-Verbindung wird vorzugsweise in einem geeigneten inerten Lösungsmittel, wie einem Kohlenwasserstoff, oder einem Aether, oder in einem Lösungsmittelgemisch vorgenommen. Je nach Reaktionsfähigkeit arbeitet man unter Kühlen oder bei erhöhter Temperatur, etwa zwischen -10° und +100°C, bevorzugt bei etwa 20° bis 80°C. Ueblicherweise arbeitet man in Gegenwart eines basischen Mittels, wie einer organischen Base, z.B. eines Amins, oder "Polystyol-Hünigbase", oder einer anorganischen Base, z.B. eines Alkalimetallcarbonats, wobei die primär entstandene Phosphoniumverbindung der Formel

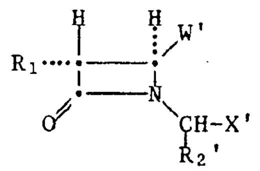

(IIa),

worin X′ eine Phosphonogruppe oder eine Phosphoniogruppe zusammen mit einem Anion, je nach Bedeutung des Restes $X_o$ (vgl. Formel VI) z.B. Chlorid, bedeutet, in das Ylid-Ausgangsmaterial der Formel II umgewandelt wird.

Die Einführung des Restes -S-C(=Z)-$R_3$ in Verbindungen der Formel II′, worin W′ für Niederalkanoylthio oder Triphenylmethylthio steht, kann in analoger Weise erfolgen, wie unter Stufe 2 beschrieben.

Man kann das im Reaktionschema I beschriebene Verfahren zur Herstellung der Verbindungen der Formeln (II), (III), (V) und (VI), sowie die angegebenen Verfahren zur Herstellung der Endprodukte der Formel (I) auch mit optisch inaktiven Verbindungen durchführen und auf einer beliebigen Verfahrensstufe aus einem erhaltenen Diastereomerengemisch oder Racemat in bekannter Weise die optisch aktiven Verbindungen gemäss der vorliegenden Erfindung isolieren.

Vorzugsweise werden solche Ausgangsverbindungen verwendet und die Reaktionsbedingungen so gewählt, dass man zu den vorstehend als besonders bevorzugt aufgeführten Verbindungen der Formel I gelangt.

Die pharmakologisch verwendbaren Verbindungen der vorliegenden Erfindung können z.B. zur Herstellung von pharmazeutischen Präparaten verwendet werden, welche eine therapeutisch wirksame Menge der Aktivsubstanz zusammen oder im Gemisch mit anorganischen oder organischen, festen oder flüssigen, pharmazeutisch annehmbaren Trägerstoffen enthalten, die sich zur oralen oder zur parenteralen, d.h. z.B. intramuskulären, intravenösen, subcutanen oder intraperitonealen, Verabreichung eignen.

Zur oralen Verabreichung verwendet man Tabletten oder Gelatinekapseln, welche den Wirkstoff zusammen mit Verdünnungsmitteln, z.B. Lactose, Dextrose, Sucrose, Mannitol, Sorbitol, Cellulose und/oder Glycin, und Schmiermitteln, z.B. Kieselerde, Talk, Stearinsäure oder Salze davon, wie Magnesium- oder Calciumstearat, und/oder Polyäthylenglykol, aufweisen. Tabletten enthalten ebenfalls Bindemittel, z.B. Magnesiumaluminiumsilikat, Stärken, wie Mais-, Weizen-, Reis-oder Pfeilwurzstärke, Gelatine, Tragacant, Methylcellulose, Natriumcarboxymethylcellulose und/oder Polyvinylpyrrolidon, und, wenn erwünscht, Sprengmittel, z.B. Stärken, Agar, Alginsäure oder Salze davon, wie Natriumalginat, und/oder Brausemischungen oder Adsorptionsmittel, Farbstoffe, Geschmackstoffe oder Süssmittel.

Zur parenteralen Verabreichung eignen sich in erster Linie Infusionslösungen, vorzugsweise isotonische wässrige Lösungen oder Suspensionen, wobei diese z.B. aus lyophilisierten Präparaten, welche die Wirksubstanz allein oder zusammen mit einem Trägermaterial, z.B. Mannit enthalten, vor Gebrauch hergestellt werden können. Solche Präparate können sterilisiert sein und/oder Hilfsstoffe, z.B. Konservier-, Stabilisier-, Netz- und-/oder Emulgiermittel, Löslichkeitsvermittler, Salze zur Regulierung des osmotischen Druckes und/oder Puffer enthalten.

Die vorliegenden pharmazeutischen Präparate, die, wenn erwünscht, weitere pharmakologisch wertvolle Stoffe enthalten können, werden in an sich bekannter Weise, z.B. mittels konventionellen Mischungs-, Lösungs- oder Lyophilisierungsverfahren, hergestellt und enthalten von etwa 0,1 % bis 100 %, insbesondere von etwa 1 % bis etwa 50 %, Lyophilisate bis zu 100 % des Aktivstoffs.

Je nach Art der Infektion und Zustand des infizierten Organismus verwendet man tägliche Dosen (oral oder parenteral) von etwa 200 mg bis etwa 1 g zur Behandlung von Warmblütern (Menschen und Tiere) von etwa 70 kg Gewicht.

Die folgenden Beispiele dienen zur Illustration der Erfindung. Temperaturen werden in Celsiusgraden angegeben.

Experimenteller Teil

Beispiel 1: (5R,6S)-2-(3-Pyridyl)-6-[(1R)-1-allyloxycarbonyloxyäthyl]-2-penem-3-carbonsäureallylester

Eine Lösung von 3,1 g 2-[(3S,4R)-3-[(1R)-1-Allyloxycarbonyloxyäthyl]-4-nicotinoylthio-2-oxo-azetidin-1-yl]-2-triphenylphosphoranylidenessigsäureallylester in 600 ml Toluol wird unter Argonatmosphäre 3 Stunden bei Rückflusstemperatur gerührt. Dann wird das Lösungsmittel abgedampft und das Rohprodukt durch Chromatographie an Silicagel gereinigt. (Laufmittel Toluol/Aethylacetat 9:1 bis 85:15)
$R_f$ (Aethylacetat): 0,5
IR ($CH_2Cl_2$): 1795; 1747; 1715; 1575 $cm^{-1}$.

Das Ausgangsmaterial kann wie folgt hergestellt werden:

2-[(3S,4R)-3-[(1R)-1-Allyloxycarbonyloxyäthyl]-4-nicotinoylthio-2-oxo-azetidin-1-yl]-2-triphenylphosphoranylidenessigsäureallylester

6,96 g Silbersalz des 2-[(3S,4R)-3-[(1R)-1-Allyloxycarbonyloxyäthyl]-4-mercapto-2-oxo-azetidin-1-yl]-2-triphenylphosphoranylidenessigsäureallylesters werden in 110 ml Methylenchlorid gelöst, bei 0° mit 3,2 ml Pyridin, 200 mg 4-Dimethylaminopyridin und anschliessend mit 2,68 g Nicotinoylchlorid-Hydrochlorid versetzt. Nach 20 Minuten Rühren bei 0° wird der Feststoff über Hyflo abfiltriert und das Filtrat wird mit wässriger Natriumhydrogencarbonat-Lösung und dann mit gesättigter NaCl-Lösung gewaschen. Nach Trocknen über $MgSO_4$ wird das Lösungsmittel abgedampft. Der Rückstand wird durch Chromstographie an Silicagel (Laufmittel Toluol-Aethylacetat 9:1 bis 3:2) gereinigt.
DC (Aethylacetat) $R_f$ = 0,33
IR ($CH_2Cl_2$): 1755; 1670; 1645; 1585 $cm^{-1}$.

Beispiel 2: (5R,6S)-2-(3-Pyridyl)-6-[(1R)-1-hydroxyäthyl]-2-penem-3-carbonsäure, Natriumsalz

Eine Lösung von 1,1 g (5R,6S)-2-(3-Pyridyl)-6-[(1R)-1-Allyloxycarbonyloxyäthyl]-2-penem-3-carbonsäureallylester in 48 ml THF wird mit 110 mg Tetrakis-(triphenylphosphin)-palladium und 1,6 ml Tributylzinnhydrid versetzt. Nach 35 Minuten Rühren bei Raumtemperatur wird das Gemisch mit 0,36 ml Essigsäure tropfenweise versetzt und weitere 30 Minuten gerührt. Die Lösung wird am Rotationsverdampfer konzentriert. Der Rückstand wird in Wasser-Aethylacetat aufgenommen und mittels $NaHCO_3$ neutral gestellt. Die wässrige Phase wird zweimal mit Aethylacetat nachgewaschen und am Hochvakuum konzentriert. Die Reinigung durch Chromatographie an Opti $UPC_{12}$ (Laufmittel Wasser) ergibt die Titelsubstanz.
DC (Opti $UPC_{12}$; Wasser) $R_f$ = 0,61
IR (DMSO-$d_6$); 1772; 1620 $cm^{-1}$.

Beispiel 3: (5R,6S)-2-(6-Methyl-pyrid-3-yl)-6-[(1R)-1-allyloxycarbonyloxyäthyl]-2-penem-3-carbonsäureallylester

Analog Beispiel 1 werden 0,3 g 2-[(3S,4R)-3-[(1R)-1-Allyloxycarbonyloxyäthyl]-4-(6-methylnicotinoylthio)-2-oxo-azetidin-1-yl]-2-triphenylphosphoranylidenessigsäureallylester in die Titelverbindung überführt.
IR ($CH_2Cl_2$): 1790; 1742; 1710 $cm^{-1}$.

Das Ausgangsmaterial kann wie folgt hergestellt werden:

2-[(3S,4R)-3-[(1R)-1-Allyloxycarbonyloxyäthyl]-4-(6-methylnicotinoylthio)-2-oxo-azetidin-1-yl]-2-triphenylphosphoranylidenessigsäureallylester

Analog Beispiel 1 werden 0,415 g Silbersalz des 2-[(3S,4R)-3-[(1R)-1-Allyloxycarbonyloxyäthyl]-4-mercapto-2-oxo-azetidin-1-yl]-2-triphenylphosphoranylidenessigsäureallylesters und 0,18 g 6-Methylnicotinoylchlorid-Hydrochlorid in die Titelverbindung überführt.
IR($CH_2Cl_2$): 1760; 1670; 1620; 1590 $cm^{-1}$.

Beispiel 4: (5R,6S)-2-(6-Methyl-pyrid-3-yl)-6-[(1R)-1-hydroxyäthyl]-2-penem-3-carbonsäure, Natriumsalz

Analog Beispiel 2 werden 100 mg (5R,6S)-2-(6-Methyl-pyrid-3-yl)-6-[(1R)-1-allyloxycarbonyloxyäthyl]-2-penem-3-carbonsäureallylester in die Titelverbindung überführt.
IR (DMSO-$d_6$): 1772; 1619 $cm^{-1}$.

Beispiel 5: (5R,6S)-2-(2-Chlorpyrid-3-yl)-6-[(1R)-1-allyloxycarbonyloxyäthyl]-2-penem-3-carbonsäureallylester

Analog Beispiel 1 werden 1,2 g 2-[(3S,4R)-3-[(1R)-1-Allyloxycarbonyloxyäthyl]-4-(2-chlornicotinoylthio)-2-oxo-azetidin-1-yl]-2-triphenylphosphoranylidenessigsäureallylester in die Titelverbindung überführt.
IR ($CH_2Cl_2$): 1795; 1750; 1720; 1570 $cm^{-1}$.

Das Ausgangsmaterial kann wie folgt hergestellt werden:

2-[(3S,4R)-3-[(1R)-1-Allyloxycarbonyloxyäthyl]-4-(2-chlornicotinoylthio)-2-oxo-azetidin-1-yl]-2-triphenylphosphoranylidenessigsäureallylester

Analog Beispiel 1 werden 1,74 g Silbersalz des 2-[(3S,4R)-3-[(1R)-1-Allyloxycarbonyloxyäthyl]-4-mercapto-2-oxo-azetidin-1-yl]-2-triphenylphosphoranylidenessigsäureallylesters und 0,6 g 2-Chlornicotinsäurechlorid in die Titelverbindung überführt.
IR ($CH_2Cl_2$): 1760; 1685; 1610; 1570 $cm^{-1}$.

Beispiel 6: (5R,6S)-2-(2-chlorpyrid-3-yl)-6-[(1R)-1-hydroxyäthyl]-2-penem-3-carbonsäure, Natriumsalz

Analog Beispiel 2 werden 0,23 g (5R,6S)-2-(2-Chlor-pyrid-3-yl)-6-[(1R)-1-allyloxycarbonyloxyäthyl]-2-penem-3-carbonsäureallylester in die Titelverbindung überführt.
IR (DMSO-$d_6$): 1775, 1620 $cm^{-1}$.

Beispiel 7: (5R,6S)-2-(3-Pyridyl)-6-[(1R)-1-hydroxyäthyl]-2-penem-3-carbonsäure-pivaloyloxymethylester

62 mg (5R,6S)-2-(3-Pyridyl)-6-[(1R)-1-hydroxyäthyl]-2-penem-3-carbonsäure, Natriumsalz werden in 1,5 ml abs. DMF gelöst, auf 0° abgekühlt und mit 53 µl Jodmethylpivalat versetzt. Nach 1 stunde Rühren wird das Reaktionsgemisch mit Aethylacetat verdünnt, dreimal mit gesätigter NaCl-Lösung gewaschen, getrocknet und eingeengt. Durch säulenchromatographie an Silicagel (Laufmittel Toluol/Aethylacetat 3:1 bis 1:1) erhält man die Titelverbindung. $R_f$ (Aethylacetat): 0,34
IR ($CH_2Cl_2$): 3600; 1790; 1750; 1730; 1570 $cm^{-1}$.
UV (Aethanol) $\lambda_{max}$: 340 nm.

Beispiel 8: (5R,6S)-2-(1-Methyl-3-pyridinio)-6-[(1R)-1-hydroxyäthyl]-2-penem-3-carboxylat

Eine Lösung von 32 mg (5R,6S)-2-(3-Pyridyl)-6-[(1R)-1-hydroxyäthyl]-2-penem-3-carbonsäure, Natriumsalz in 1,2 ml Wasser und 1,2 ml Dioxan wird bei pH 8 mit 58 µl Dimethylsulfat versetzt. Nach 45 Minuten wird das Reaktionsgemisch zweimal mit Aether gewaschen. Nach Einstellen des pH auf 7,5 wird die Lösung am Hochvakuum konzentriert und an OPTI $UPC_{12}$ chromatographiert (Laufmittel: Wasser).
$R_f$ (Wasser/Acetonitril 4:1): 0,54
IR (DMSO-$d_6$): 1776; 1624 $cm^{-1}$
UV (Wasser) $\lambda_{max}$: 336 nm

Beispiel 9: (5R,6S)-2-(1-Methoxycarbonylmethyl-3-pyridinio)-6-[(1R)-hydroxyäthyl]-2-penem-3-carboxylat

31 mg (5R,6S)-2-(3-Pyridyl)-6-[(1R)-1-hydroxyäthyl]-2-penem-3-carbonsäure, Natriumsalz werden in 0,3 ml Wasser gelöst und mit einer Lösung von 9,3 µl Bromessigsäuremethylester in 0,1 ml Aceton versetzt. Nach 2 Stunden Rühren bei Raumtemperatur und weiteren 6 Stunden bei 35° wird mit Wasser verdünnt und durch

Chromatographie auf Opti $UPC_{12}$ (Laufmittel $H_2O$) und die Titelsubstanz erhalten.
DC (Opti $UPC_{12}$; Wasser/Acetonitril 4:1) $R_f$ = 0,38;
UV (Wasser) $\lambda_{max}$ = 333 nm
IR (DMSO-$d_6$): 3433; 1776; 1752; 1624 $cm^{-1}$

Beispiel 10: (5R,6S)-2-(3-Pyridil)-6-[(1R)-1-hydroxyäthyl]-2-penem-3-carbonsäure-1-äthoxycarbonyloxyäthylester

1,2 g Natriumjodid werden in 3,7 ml Aceton gelöst und mit 0,275 ml Aethyl-1-chloräthylcarbonat versetzt. Das Gemisch wird bei Raumtemperatur während 3 Stunden gerührt. Anschliessend wird die Lösung auf 10,5 ml Methylenchlorid getropft und von den ausgefallenen anorganischen Salzen abfiltriert. Die Methylenchloridlösung wird bis auf 2 ml eingeengt und bei 0° zu einer Lösung von 0,314 g (1 mMol) (5R,6S)-2-(3-Pyridyl)-6-[(1R)-1-hydroxyäthyl]-2-penem-3-carbonsäure in 4 ml Dimethylacetamid gegeben. Dann wird während 3 Stunden bei 0° gerührt, anschliessend mit Aethylacetat verdünnt und dreimal mit Wasser gewaschen. Die organischen Phasen werden über Natriumsulfat getrocknet und am Rotationsverdampfer eingeengt. Das Rohprodukt wird an 10 g Silicagel mit dem Laufmittel Aethylacetat gereinigt. Man erhält die Titelverbindung als weissen Schaum. IR-Spektrum (Methylenchlorid): 3600; 1790; 1755; 1722; 1670 $cm^{-1}$.
UV (Aethanol) $\lambda_{max}$ : 341 nm.

Beispiel 11: (5R,6S)-2-(3-Pyridyl)-6-[(1R)-1-allyloxycarbonyloxyäthyl]-2-penem-3-carbonsäureallylester

Eine Lösung von 4,37 g (3S,4R)-3-[(1R)-1-Allyloxycarbonyloxyäthyl]-4-nicotinoylthio-azetidin-2-on in 45 ml trockenem Methylenchlorid wird nacheinander bei -20° mit 1,76 ml Allyloxalylchlorid und 2,43 ml Hünig-Base versetzt. Nach 25 Minuten Rühren bei -20° wird das Reaktionsgemisch auf 50 ml einer pH 7-Pufferlösung gegossen, und die organische Phase nach Waschen mit gesättigter Kochsalz-Lösung getrocknet und eingeengt. Der so erhaltene rohe Oxalsäureamidester wird in 24 ml destilliertem Triäthylphosphit während 135 Minuten bei Raumtemperatur gerührt, anschliessend mit 25 ml olefinfreiem Decan versetzt und am Hochvakuum eingeengt. Dieses Einengen mit Decan wird zweimal wiederholt. Das rohe Phosphoran wird in 40 ml absolutem Toluol gelöst und über Nacht bei 110° unter Argon gerührt. Nach Einengen wird die Titelverbindung durch Chromatographie an Silicagel (Laufmittel Toluol bis Toluol/Aethylacetat 95:5) gereinigt.
DC (Aethylacetat) $R_f$ = 0,50
IR ($CH_2Cl_2$): 1795; 1747; 1715; 1575 $cm^{-1}$.

Die so erhaltene geschütze Penem-Verbindung wird, wie in Beispiel 2 beschrieben, in das Natriumsalz der (5R,6S)-2-(3-Pyridyl)-6-[(1R)-1-hydroxyäthyl]-2-penem-3-carbonsäure überführt.

Das Ausgangsmaterial wird wie folgt hergestellt:

(3S,4R)-3-[(1R)-1-Allyloxycarbonyloxyäthyl]-4-nicotinoylthioazetidin-2-on

7 g (3S,4R)-3-[(1R)-1-Allyloxycarbonyloxyäthyl]-4-acetoxy-azetidin-2-on (vgl. Europäische Patentanmeldung Nr. 126709) und 4,2 g Thionicotinsäure werden in 180 ml Methylenchlorid gelöst. Diese Lösung wird zuerst mit 180 ml Wasser und dann mit 30 ml 1 N NaOH-Lösung versetzt. Die kräftig gerührte Emulsion wird 1,5 h bei Raumtemperatur gerührt. Dann wird die organische Phase abgetrennt und die wässrige Phase zweimal mit Methylenchlorid extrahiert. Die vereinigten organischen Extrakte werden mit einer gesättigten wässrigen $NaHCO_3$-Lösung und dann mit gesättigter Kochsalz-Lösung gewaschen, über $MgSO_4$ getrocknet und eingedampft. Das erhaltene Rohprodukt wird durch Chromatographie an Silicagel mit Toluol/Aethylacetat gereinigt.
IR ($CH_2Cl_2$); 3400; 1780; 1743; 1670 $cm^{-1}$.

Beispiel 12: (5R,6S)-2-(3-Pyridyl)-6-[(1R)-1-hydroxyäthyl]-2-penem-3-carbonsäure-acetoxymethylester

63,1 mg (5R,6S)-2-(3-Pyridyl)-6-[(1R)-1-hydroxyäthyl]-2-penem-3-carbonsäure-Natriumsalz werden in 2 ml abs. DMF und 0,2 ml abs. DMSO gelöst und bei 0° unter Rühren tropfenweise mit einer Lösung von 39,8 mg Acetoxybrommethan in 0,3 ml abs. DMF versetzt. Nach 30 Minuten Rühren bei 0° und anschliessend 30 Minuten bei Raumtemperatur wird das Reaktionsgemisch mit Aethylacetat verdünnt und zweimal mit gesättigter Kochsalz-Lösung gewaschen. Die organische Phase wird über Magnesiumsulfat getrocknet und eingedampft. Die Reinigung durch Säulenchromatographie (Laufmittel Aethylacetat) ergibt die Titelverbindung. IR (Methylenchlorid): 3585; 1792; 1765; 1728; 1580 $cm^{-1}$; UV (Aethanol) $\lambda_{max}$ = 339 nm.

Beispiel 13: In analoger Weise, wie in den vorangehenden Beispielen beschrieben, können die folgenden Verbindungen hergestellt werden:

(5R,6S)-6-[(1R)-1-Hydroxyäthyl)-2-(6-methoxycarbonyl-pyrid-3-yl)-2-penem-3-carbonsäure, Natriumsalz, UV (Wasser) $\lambda_{max}$ 325 nm;
IR (DMSO-$d_6$): 1771 $cm^{-1}$.

(5R,6S)-6-[(1R)-1-Hydroxyäthyl)-2-(2-methoxycarbonyl-pyrid-3-yl)-2-penem-3-carbonsäure, Natriumsalz, UV (Wasser) $\lambda_{max}$ 315 nm;
IR (DMSO-$d_6$): 1773 $cm^{-1}$.

(5R,6S)-6-[(1R)-1-Hydroxyäthyl)-2-(6-hydroxy-pyrid-3-yl)-2-penem-3-carbonsäure, Natriumsalz, UV (Wasser) $\lambda_{max}$ 323 nm;
IR (DMSO-$d_6$): 1772 $cm^{-1}$.

(5R,6S)-6-[(1R)-1-Hydroxyäthyl)-2-(6-hydroxymethyl-pyrid-3-yl)-2-penem-3-carbonsäure, Natriumsalz, UV (Wasser) $\lambda_{max}$ 325 nm;
IR (DMSO-$d_6$): 1773 $cm^{-1}$.

(5R,6S)-6-[(1R)-1-Hydroxyäthyl)-2-(2-methylthio-pyrid-3-yl)-2-penem-3-carbonsäure, Natriumsalz, UV (Wasser) $\lambda_{max}$ 310 nm;
IR (DMSO-$d_6$): 1765 $cm^{-1}$.

(5R,6S)-2-(6-Chlor-pyrid-3-yl)-6-[(1R)-1-hydroxyäthyl]-2-penem-3-carbonsäure, Natriumsalz, UV (Wasser) $\lambda_{max}$ 328 nm;
IR (DMSO-$d_6$): 1770 $cm^{-1}$.

(5R,6S)-2-(2-Amino-pyrid-3-yl)-6-[(1R)-1-hydroxyäthyl]-2-penem-3-carbonsäure, Natriumsalz, UV (Wasser) $\lambda_{max}$ 312 nm;
IR (DMSO-$d_6$): 1768 $cm^{-1}$.

(5R,6S)-2-(6-Amino-pyrid-3-yl)-6-[(1R)-1-hydroxyäthyl]-2-penem-3-carbonsäure, Natriumsalz, UV (Wasser) $\lambda_{max}$ 325 nm;
IR (DMSO-$d_6$): 1771 $cm^{-1}$.

(5R,6S)-2-(4-Carbamoyl-pyrid-3-yl)-6-[(1R)-1-hydroxyäth1l]-2-penem-3-carbonsäure, Natriumsalz, UV (Wasser) $\lambda_{max}$ 318 nm;
IR (DMSO-$d_6$): 1773 $cm^{-1}$.

(5R,6S)-2-(2-Carbamoyl-pyrid-3-yl)-6-[(1R)-1-hydroxyäthyl]-2-penem-3-carbonsäure, Natriumsalz, UV (Wasser) $\lambda_{max}$ 315 nm;
IR (DMSO-$d_6$): 1770 $cm^{-1}$.

(5R,6S)-2-(6-Aminomethyl-pyrid-3-yl)-6-[(1R)-1-hydroxyäthyl]-2-penem-3-carbonsäure, Natriumsalz, UV (Wasser) $\lambda_{max}$ 327 nm;
IR (DMSO-$d_6$): 1771 $cm^{-1}$.

(5R,6S)-2-(6-Carbamoyloxymethyl-pyrid-3-yl)-6-[(1R)-1-hydroxyäthyl]-2-penem-3-carbonsäure, Natriumsalz, UV (Wasser) $\lambda_{max}$ 324 nm;
IR (DMSO-$d_6$): 1772 $cm^{-1}$.

(5R,6S)-2-[6-(2-Aminoäthoxy)-pyrid-3-yl)-6-[(1R)-1-hydroxyäthyl]-2-penem-3-carbonsäure, Natriumsalz, UV (Wasser) $\lambda_{max}$ 322 nm;
IR (DMSO-$d_6$): 1774 $cm^{-1}$.

(5R,6S)-2-(2-Carboxy-pyrid-3-yl)-6-[(1R)-1-hydroxyäthyl]-2-penem-3-carbonsäure, Dinatriumsalz, UV (Wasser) $\lambda_{max}$ 313 nm;
IR (DMSO-$d_6$): 1771 $cm^{-1}$.

(5R,6S)-2-(4-Carboxy-pyrid-3-yl)-6-[(1R)-1-hydroxyäthyl]-2-penem-3-carbonsäure, Dinatriumsalz, UV (Wasser) $\lambda_{max}$ 315 nm;
IR (DMSO-$d_6$): 1772 $cm^{-1}$.

(5R,6S)-2-(1-Carbamoylmethyl-3-pyridinio)-6-[(1R)-1-hydroxyäthyl]-2-penem-3-carboxylat, UV (Wasser) $\lambda_{max}$ 333 nm;
IR (DMSO-$d_6$): 3435; 1776; 1700; 1623 $cm^{-1}$.

(5R,6S)-2-(1-Carboxylatomethyl-3-pyridinio)-6-[(1R)-1-hydroxyäthyl]-2-penem-3-carbonsäure, Natriumsalz, IR (KBr): 1769; 1630; 1375 $cm^{-1}$.

(5R,6S)-2-(1-Cyanomethyl-3-pyridinio)-6-[(1R)-1-hydroxyäthyl]-2-penem-3-carboxylat, UV (Wasser) $\lambda_{max}$ 332 nm.

(5R,6S)-2-(4-Methoxycarbonyl-pyrid-3-yl)-6-[(1R)-1-hydroxyäthyl]-2-penem-3-carbonsäure, Natriumsalz UV (Wasser) $\lambda_{max}$ : 278 nm;
IR (DMSO-$d_6$): 3437; 1773; 1733; 1618 $cm^{-1}$.

(5R,6S)-2-(4-Cyanoäthoxycarbonyl-pyrid-3-yl)-6-[(1R)-1-hydroxyäthyl]-2-penem-3-carbonsäure
UV (Wasser) $\lambda_{max}$ : 280 nm;

Beispiel 14: Trockenampullen oder Vials, enthaltend 0,5 g (5R,6S)-2-(3-Pyridyl)-6-[(1R)-1-hydroxyäthyl]-2-penem-3-carbonsäure, Natriumsalz als Wirksubstanz, werden wie folgt hergestellt:

Zusammensetzung (für 1 Ampulle oder Vial):

| | |
|---|---|
| Wirksubstanz | 0,5 g |
| Mannit | 0,5 g |

Eine sterile wässrige Lösung der Wirksubstanz und des Mannits wird unter aseptischen Bedingungen in 5 ml-Ampullen oder 5 ml-Vials der Gefriertrocknung unterworfen und die Ampullen bzw. Vials verschlossen und geprüft.

Anstelle des obengenannten Wirkstoffs kann euch dieselbe Menge eines anderen Wirkstoffs der vorangehenden Beispiele verwendet werden.

## Patentansprüche

### Patentansprüche für folgende Vertragsstaaten : BE, CH, DE, FR, GB, IT, LI, LU, NL, SE

1. Verbindungen der Formel

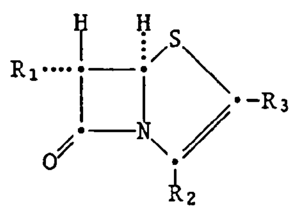

(I),

worin $R_1$ Hydroxymethyl oder 1-Hydroxyäthyl ist, $R_2$ Carboxyl oder unter physiologischen Bedingungen spaltbares verestertes Carboxyl bedeutet; und $R_3$ 3-Pyridyl oder durch Hydroxy, Niederalkoxy, Aminoniederalkoxy, Niederalkanoyloxy, Halogen, Mercapto, Niederalkylthio, Aminoniederalkylthio, Phenylthio, Niederalkyl, Hydroxyniederalkyl, Niederalkanoyloxyniederalkyl, Carboxyniederalkyl, Niederalkoxycarbonylniederalkyl, durch Cyano oder Amino substituiertes Niederalkoxycarbonylniederalkyl, Carbamoylniederalkyl, Carbamoyloxyniederalkyl, Cyanoniederalkyl, Halogenniederalkyl, Aminoniederalkyl, Niederalkylaminoniederalkyl, Diniederalkylaminoniederalkyl, Niederalkanoylaminoniederalkyl, Amino-carboxyniederalkyl, Sulfoniederalkyl, Amino, Niederalkylamino, Diniederalkylamino, Niederalkanoylamino, Carboxyl, Niederalkoxycarbonyl, durch Cyano oder Amino substituiertes Niederalkoxycarbonyl, Carbamoyl, Cyano, Sulfo, Sulfamoyl, Oxido und/oder gegebenenfalls durch Niederalkyl, Niederalkoxy und/oder Halogen substituiertes Phenyl substituiertes 3-Pyridyl darstellt, worin mit "nieder" bezeichnete Reste 1 bis 7 Kohlenstoffatome enthalten, und Salze von Verbindungen der Formel I.

2. Verbindungen der Formel I gemäss Anspruch 1, worin $R_1$ Hydroxymethyl oder 1-Hydroxyäthyl ist, $R_2$ Carboxyl oder unter physiologischen Bedingungen spaltbares verestertes Carboxyl bedeutet und $R_3$ 3-Pyridyl oder durch Hydroxy, Niederalkoxy, Aminoniederalkoxy, Halogen, Niederalkyl, Hydroxyniederalkyl, Carboxyniederalkyl, Niederalkoxycarbonylniederalkyl, Carbamoyloxyniederalkyl, Carbamoylniederalkyl, Aminoniederalkyl, Cyanoniederalkyl, Amino, Carboxy, Niederalkoxycarbonyl oder Carbamoyl substituiertes 3-Pyridyl bedeutet, worin mit "nieder" bezeichnete Reste 1 bis 7 Kohlenstoffatome enthalten, und Salze von Verbindungen der Formel I.

3. Verbindungen der Formel I gemäss Anspruch 1, worin $R_1$ Hydroxymethyl oder 1-Hydroxyäthyl ist, $R_2$ Carboxyl oder unter physiologischen Bedingungen spaltbares verestertes Carboxyl bedeutet und $R_3$ 3-Pyridyl oder durch Niederalkyl oder Halogen substituiertes 3-Pyridyl darstellt, worin mit "nieder" bezeichnete Reste 1 bis 7 Kohlenstoffatome enthalten, und Salze von Verbindungen der Formel I.

4. Verbindungen der Formel I gemäss Anspruch 1, worin $R_1$ (1R)-1-Hydroxyäthyl ist, und $R_2$ und $R_3$ die in Anspruch 1 angegebenen Bedeutungen haben, und Salze von Verbindungen der Formel I.

5. Pharmazeutisch annehmbare Salze von Verbindungen der Formel I gemäss Anspruch 1.

6. (5R,6S)-2-(3-Pyridyl)-6-[(1R)-1-hydroxyäthyl]-2-penem-3-carbonsäure und pharmazeutisch annehmbare Salze davon gemäss Anspruch 1.

7. (5R,6S)-2-(6-Methyl-pyrid-3-yl)-6-[(1R)-1-hydroxyäthyl]-2-penem-3-carbonsäure und pharmazeutisch annehmbare Salze davon gemäss Anspruch 1.

8. (5R,6S)-2-(2-Chlorpyrid-3-yl)-6-[(1R)-1-hydroxyäthyl]-2-penem-3-carbonsäure und pharmazeutisch annehmbare Salze davon gemäss Anspruch 1.

9. (5R,6S)-2-(6-Amino-pyrid-3-yl)-6-[(1R)-1-hydroxyäthyl]-2-penem-3-carbonsäure und pharmazeutisch annehmbare Salze davon gemäss Anspruch 1.

10. Unter physiologischen Bedingungen spaltbare Ester der (5R,6S)-2-(3-Pyridyl)-6-[(1R)-1-hydroxyäthyl]-2-penem-3-carbonsäure gemäss Anspruch 1.

11. (5R,6S)-2-(3-Pyridyl)-6-[(1R)-1-hydroxyäthyl]-2-penem-3-carbonsäure-1-äthoxycarbonyloxyäthylester gemäss Anspruch 1.

12. (5R,6S)-2-(3-Pyridyl)-6-[(1R)-1-hydroxyäthyl]-2-penem-3-carbonsäure-acetoxymethylester gemäss Anspruch 1.

13. (5R,6S)-2-(3-Pyridyl)-6-[(1R)-1-hydroxyäthyl]-2-penem-3-carbonsäure-pivaloyloxymethylester gemäss Anspruch 1.

14. Eine Verbindung gemäss Anspruch 1 und ihre pharmazeutisch annehmbaren Salze zur Anwendung in einem Verfahren zur therapeutischen Behandlung des menschlichen und tierischen Körpers.

15. Pharmazeutisches Präparat enthaltend eine Verbindung der Formel I gemäss Anspruch 1 oder ein pharmazeutisch annehmbares Salz einer solchen Verbindung mit salzbildender Gruppe.

16. Verwendung von Verbindungen der Formel I gemäss Anspruch 1 und von pharmazeutisch annehmbaren Salzen von solchen Verbindungen mit salzbildenden Gruppen zur Herstellung von pharmazeutischen Präparaten.

17. Verfahren zur Herstellung von Verbindungen der Formel I gemäss Anspruch 1, dadurch gekennzeichnet, dass man eine Ylid-Verbindung der Formel

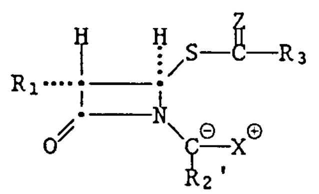

(II),

worin $R_1$ und $R_3$ die unter Formel I angegebenen Bedeutungen haben, worin die Hydroxygruppe im Rest $R_1$ sowie weitere im Rest $R_3$ enthaltene funktionelle Gruppen durch konventionelle Schutzgruppen geschützt sind, $R_2'$ eine geschützte Carboxylgruppe darstellt, Z Sauerstoff oder Schwefel bedeutet und $X^{\oplus}$ entweder eine dreifach substituierte Phosphoniogruppe oder eine zweifach veresterte Phosphonogruppe zusammen mit einem Kation darstellt, ringschliesst, oder eine Verbindung der Formel

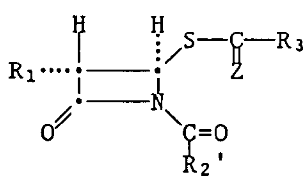

(III),

worin $R_1$ und $R_3$ die unter Formel I und $R_2'$ und Z die unter Formel II angegebenen Bedeutungen haben, worin die Hydroxygruppe im Rest $R_1$ sowie weitere im Rest $R_3$ enthaltene funktionelle Gruppen durch konventionelle Schutzgruppen geschützt sind, mit einer organischen Verbindung des dreiwertigen Phosphors behandelt, und in einer erhältlichen Verbindung geschützte funktionelle Gruppen in die freien funktionellen Gruppen überführt, und, wenn erwünscht, in einer erhältlichen Verbindung der Formel I eine freie Carboxylgruppe $R_2$ in eine unter physiologischen Bedingungen spaltbare veresterte Carboxylgruppe $R_2$ überführt, und/oder, wenn erwünscht, in einer erhältlichen Verbindung der Formel I einen Rest $R_3$ in einen anderen Rest $R_3$ überführt, und/oder, wenn erwünscht, eine erhältliche Verbindung mit salzbildender Gruppe in ein Salz oder ein erhältliches Salz in die freie Verbindung oder in ein anderes Salz überführt.

18. Die nach dem Verfahren gemäss Anspruch 17 erhältlichen Verbindungen.

**Patentansprüche für folgende Vertragsstaaten : AT, ES, GR**

1. Verfahren zur Herstellung von Verbindungen der Formel

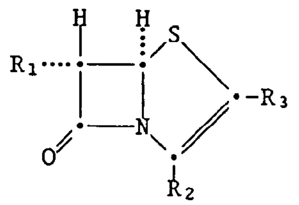

(I),

worin $R_1$ Hydroxymethyl oder 1-Hydroxyäthyl ist, $R_2$ Carboxyl oder unter physiologischen Bedingungen spaltbares verestertes Carboxyl bedeutet; und $R_3$ 3-Pyridyl oder durch Hydroxy, Niederalkoxy, Aminoniederalkoxy, Niederalkanoyloxy, Halogen, Mercapto, Niederalkylthio, Aminoniederalkylthio, Phenylthio, Niederalkyl, Hydroxyniederalkyl, Niederalkanoyloxyniederalkyl, Carboxyniederalkyl, Niederalkoxycarbonylniederalkyl, durch Cyano oder Amino substituiertes Niederalkoxycarbonylniederalkyl, Carbamoylniederalkyl, Carbamoyloxyniederalkyl, Cyanoniederalkyl, Halogenniederalkyl, Aminoniederalkyl, Niederalkylaminoniederalkyl, Diniederalkylaminoniederalkyl, Niederalkanoylaminoniederalkyl, Amino-carboxyniederalkyl, Sulfoniederalkyl, Amino, Niederalkylamino, Diniederalkylamino, Niederalkanoylamino, Carboxyl, Niederalkoxycarbonyl, durch Cyano oder Amino substituiertes Niederalkoxycarbonyl, Carbamoyl, Cyano, Sulfo, Sulfamoyl, Oxido und/oder gegebenenfalls durch Niederalkyl, Niederalkoxy und/oder Halogen substituiertes Phenyl substituiertes 3-Pyridyl darstellt, worin mit "nieder" bezeichnete Reste 1 bis 7 Kohlenstoffatome enthalten, und Salzen von Verbindungen der Formel I, dadurch gekennzeichnet, dass man eine Ylid-Verbindung der Formel

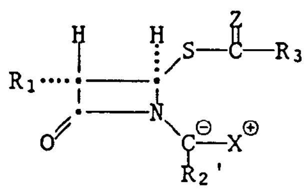

(II),

worin $R_1$ und $R_3$ die unter Formel I angegebenen Bedeutungen haben, worin die Hydroxygruppe im Rest $R_1$ sowie weitere im Rest $R_3$ enthaltene funktionelle Gruppen durch konventionelle Schutzgruppen geschützt sind, $R_2'$ eine geschützte Carboxylgruppe darstellt, Z Sauerstoff oder Schwefel bedeutet und $X^{\oplus}$ entweder eine dreifach substituierte Phosphoniogruppe oder eine zweifach veresterte Phosphonogruppe zusammen mit einem Kation darstellt, ringschliesst, oder eine Verbindung der Formel

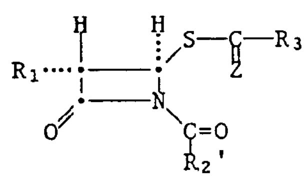

(III),

worin $R_1$ und $R_3$ die unter Formel I und $R_2'$ und Z die unter Formel II angegebenen Bedeutungen haben, worin die Hydroxygruppe im Rest $R_1$ sowie weitere im Rest $R_3$ enthaltene funktionelle Gruppen durch konventionelle Schutzgruppen geschützt sind, mit einer organischen Verbindung des dreiwertigen Phosphors behandelt, und in einer erhältlichen Verbindung geschützte funktionelle Gruppen in die freien funktionellen Gruppen überführt, und, wenn erwünscht, in einer erhältlichen Verbindung der Formel I eine freie Carboxylgruppe $R_2$ in eine unter physiologischen Bedingungen spaltbare veresterte Carboxylgruppe $R_2$ überführt, und/oder, wenn erwünscht, in einer erhältlichen Verbindung der Formel I einen Rest $R_3$ in einen anderen Rest $R_3$ überführt, und/oder, wenn erwünscht, eine erhältliche Verbindung mit salzbildender Gruppe in ein Salz oder ein erhältliches Salz in die freie Verbindung oder in ein anderes Salz überführt.

2. Verfahren zur Herstellung von Verbindungen der Formel I gemäss Anspruch 1, worin $R_1$ Hydroxymethyl oder 1-Hydroxyäthyl ist, $R_2$ Carboxyl oder unter physiologischen Bedingungen spaltbares verestertes Carboxyl bedeutet und $R_3$ 3-Pyridyl oder durch Hydroxy, Niederalkoxy, Aminoniederalkoxy, Halogen, Niederalkyl, Hydroxyniederalkyl, Carboxyniederalkyl, Niederalkoxycarbonylniederalkyl, Carbamoyloxyniederalkyl, Carbamoylniederalkyl, Aminoniederalkyl, Cyanoniederalkyl, Amino, Carboxy, Niederalkoxycarbonyl oder Carbamoyl substituiertes 3-Pyridyl bedeutet, worin mit "nieder" bezeichnete Reste 1 bis 7 Kohlenstoffatome enthalten, und Salzen von Verbindungen der Formel I.

3. Verfahren zur Herstellung von Verbindungen der Formel I gemäss Anspruch 1, worin $R_1$ Hydroxymethyl oder 1-Hydroxyäthyl ist, $R_2$ Carboxyl oder unter physiologischen Bedingungen spaltbares verestertes Carboxyl bedeutet und $R_3$ 3-Pyridyl oder durch Niederalkyl oder Halogen substituiertes 3-Pyridyl darstellt, worin mit "nieder" bezeichnete Reste 1 bis 7 Kohlenstoffatome enthalten, und Salzen von Verbindungen der Formel I.

4. Verfahren zur Herstellung von Verbindungen der Formel I gemäss Anspruch 1, worin $R_1$ (1R)-1-Hydroxyäthyl ist und $R_2$ und $R_3$ die in Anspruch 1 angegebenen Bedeutungen haben und Salzen davon.

5. Verfahren gemäss Anspruch 1 zur Herstellung von (5R,6S)-2-(3-Pyridyl)-6-[(1R)-1-hydroxyäthyl]-2-penem-3-carbonsäure und pharmazeutisch annehmbaren Salzen davon.

6. Verfahren gemäss Anspruch 1 zur Herstellung von (5R,6S)-2-(6-Methyl-pyrid-3-yl)-6-[(1R)-1-hydroxyäthyl]-2-penem-3-carbonsäure und pharmazeutisch annehmbare Salze davon.

7. Verfahren gemäss Anspruch 1 zur Herstellung von (5R,6S)-2-(2-Chlorpyrid-3-yl)-6-[(1R)-1-hydroxyäthyl]-2-penem-3-carbonsäure und pharmazeutisch annehmbare Salze davon.

8. Verfahren gemäss Anspruch 1 zur Herstellung von (5R,6S)-2-(6-Amino-pyrid-3-yl)-6-[(1R)-1-hydroxyäthyl]-2-penem-3-carbonsäure und pharmazeutisch annehmbaren Salzen davon.

9. Verfahren gemäss Anspruch 1 zur Herstellung von unter physiologischen Bedingungen spaltbaren Estern der (5R,6S)-2-(3-Pyridyl)-6-[(1R)-1-hydroxyäthyl]-2-penem-3-carbonsäure.

10. Verfahren gemäss Anspruch 1 zur Herstellung von (5R,6S)-2-(3-Pyridyl)-6-[(1R)-1-hydroxyäthyl]-2-penem-3-carbonsäure-1-äthoxycarbonyloxyäthylester.

11. Verfahren gemäss Anspruch 1 zur Herstellung von (5R,6S)-2-(3-Pyridyl)-6-[(1R)-1-hydroxyäthyl]-2-penem-3-carbonsäure-acetoxymethylester.

12. Verfahren gemäss Anspruch 1 zur Herstellung von (5R,6S)-2-(3-Pyridyl)-6-[(1R)-1-hydroxyäthyl]-2-penem-3-carbonsäure-pivaloyloxymethylester.

13. Verfahren zur Herstellung von pharmazeutischen Präparaten, dadurch gekennzeichnet, dass man eine nach dem Verfahren gemäss Anspruch 1 erhältliche Verbindung der Formel I oder ein pharmazeutisch verwendbares Salz einer solchen Verbindung mit einem pharmazeutisch verwendbaren Trägermaterial mischt.

**Claims**

**Claims for the following Contracting States : BE, CH, DE, FR, GB, IT, LI, LU, NL, SE**

1. A compound of the formula

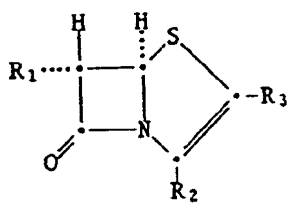

(I),

in which $R_1$ represents hydroxymethyl or 1-hydroxyethyl, $R_2$ represents carboxy or esterified carboxy that can be cleaved under physiological conditions, and $R_3$ represents 3-pyridyl or 3-pyridyl substituted by hydroxy, lower alkoxy, amino-lower alkoxy, lower alkanoyloxy, halogen, mercapto, lower alkylthio, amino-lower alkylthio, phenylthio, lower alkyl, hydroxy-lower alkyl, lower alkanoyloxy-lower alkyl, carboxy-lower alkyl, lower alkoxycarbonyl-lower alkyl, cyano- or amino-substituted lower alkoxycarbonyl-lower alkyl, carbamoyl-lower alkyl, carbamoyloxy-lower alkyl, cyano-lower alkyl, halo-lower alkyl, amino-lower alkyl, lower alkylamino-lower alkyl, di-lower alkylamino-lower alkyl, lower alkanoylamino-lower alkyl, amino-carboxy-lower alkyl, sulpho-lower alkyl, amino, lower alkylamino, di-lower alkylamino, lower alkanoylamino, carboxy, lower alkoxycarbonyl, cyano- or amino-substituted lower alkoxycarbonyl, carbamoyl, cyano, sulpho, sulphamoyl, oxido and/or by optionally lower alkyl-, lower alkoxy- and/or halosubstituted phenyl, wherein radicals designated "lower" contain from 1 to 7 carbon atoms, or a salt of a compound of the formula I.

2. A compound of the formula I according to claim 1 in which $R_1$ represents hydroxymethyl or 1-hydroxyethyl, $R_2$ represents carboxy or esterified carboxy that can be cleaved under physiological conditions, and $R_3$ represents 3-pyridyl or 3-pyridyl substituted by hydroxy, lower alkoxy, amino-lower alkoxy, halogen, lower alkyl, hydroxy-lower alkyl, carboxy-lower alkyl, lower alkoxycarbonyl-lower alkyl, carbamoyloxy-lower alkyl, carbamoyl-lower alkyl, amino-lower alkyl, cyano-lower alkyl, amino, carboxy, lower alkoxycarbonyl or by carbamoyl, wherein radicals designated "lower" contain from 1 to 7 carbon atoms, or a salt of a compound of the

formula I.

3. A compound of the formula I according to claim 1 in which $R_1$ represents hydroxymethyl or 1-hydroxyethyl, $R_2$ represents carboxy or esterified carboxy that can be cleaved under physiological conditions, and $R_3$ represents 3-pyridyl or 3-pyridyl substituted by lower alkyl or by halogen, wherein radicals designated "lower" contain from 1 to 7 carbon atoms, or a salt of a compound of the formula I.

4. A compound of the formula I according to claim 1 in which $R_1$ represents (1R)-1-hydroxyethyl, and $R_2$ and $R_3$ have the meanings given in claim 1, or a salt of a compound of the formula I.

5. A pharmaceutically acceptable salt of a compound of the formula I according to claim 1.

6. (5R,6S)-2-(3-pyridyl)-6-[(1R)-1-hydroxyethyl]-2-penem-3-carboxylic acid or a pharmaceutically acceptable salt thereof according to claim 1.

7. (5R,6S)-2-(6-methylpyrid-3-yl)-6-[(1R)-1-hydroxyethyl]-2-penem-3-carboxylic acid or a pharmaceutically acceptable salt thereof according to claim 1.

8. (5R,6S)-2-(2-chloropyrid-3-yl)-6-[(1R)-1-hydroxyethyl]-2-penem-3-carboxylic acid or a pharmaceutically acceptable salt thereof according to claim 1.

9. (5R,6S)-2-(6-aminopyrid-3-yl)-6-[(1R)-1-hydroxyethyl]-2-penem-3-carboxylic acid or a pharmaceutically acceptable salt thereof according to claim 1.

10. An ester of (5R,6S)-2-(3-pyridyl)-6-[(1R)-1-hydroxyethyl]-2-penem-3-carboxylic acid that can be cleaved under physiological conditions according to claim 1.

11. (5R,6S)-2-(3-pyridyl)-6-[(1R)-1-hydroxyethyl]-2-penem-3-carboxylic acid 1-ethoxycarbonyloxyethyl ester according to claim 1.

12. (5R,6S)-2-(3-pyridyl)-6-[(1R)-1-hydroxyethyl]-2-penem-3-carboxylic acid acetoxymethyl ester according to claim 1.

13. (5R,6S)-2-(3-pyridyl)-6-[(1R)-1-hydroxyethyl]-2-penem-3-carboxylic acid pivaloyloxymethyl ester according to claim 1.

14. A compound according to claim 1 or a pharmaceutically acceptable salt thereof for use in a method for the therapeutic treatment of the human and animal body.

15. A pharmaceutical preparation containing a compound of the formula I according to claim 1 or a pharmaceutically acceptable salt of such a compound having a salt-forming group.

16. The use of a compound of the formula I according to claim 1 or of a pharmaceutically acceptable salt of such a compound having a salt-forming group for the manufacture of a pharmaceutical preparation.

17. A process for the manufacture of a compound of the formula I according to claim 1, characterised in that an ylide compound of the formula

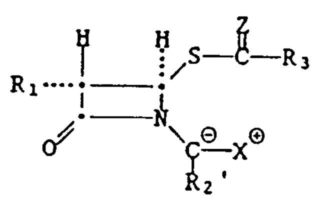

(II),

in which $R_1$ and $R_3$ have the meanings given under formula I, wherein the hydroxy group in the radical $R_1$ and other functional groups contained in the radical $R_3$ are protected by conventional protecting groups, $R_2'$ represents a protected carboxy group, Z represents oxygen or sulphur and $X^{\oplus}$ represents either a trisubstituted phosphonio group or a diesterified phosphono group together with a cation, is cyclised, or a compound of the formula

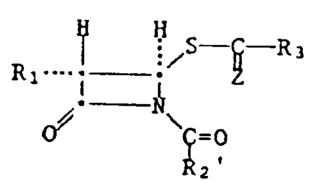

(III),

in which $R_1$ and $R_3$ have the meanings given under formula I and $R_2'$ and Z have the meanings given under formula II, wherein the hydroxy group in the radical $R_1$ and other functional groups contained in the radical $R_3$ are protected by conventional protecting groups, is treated with an organic compound of trivalent phosphorus, and in an obtainable compound protected functional groups are converted into the free functional groups, and, if desired, in an obtainable compound of the formula I a free carboxy group $R_2$ is converted into an esterified

carboxy group $R_2$ that can be cleaved under physiological conditions, and/or, if desired, in an obtainable compound of the formula I a radical $R_3$ is converted into a different radical $R_3$, and/or, if desired, an obtainable compound having a salt-forming group is converted into a salt, or an obtainable salt is converted into the free compound or into a different salt.

18. A compound obtainable in accordance with the process according to claim 17.

**Claims for the following Contracting States : AT, ES, GR**

1. A process for the manufacture of a compound of the formula

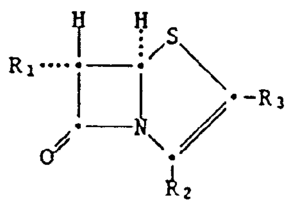

(I),

in which $R_1$ represents hydroxymethyl or 1-hydroxyethyl, $R_2$ represents carboxy or esterified carboxy that can be cleaved under physiological conditions, and $R_3$ represents 3-pyridyl or 3-pyridyl substituted by hydroxy, lower alkoxy, amino-lower alkoxy, lower alkanoyloxy, halogen, mercapto, lower alkylthio, amino-lower alkylthio, phenylthio, lower alkyl, hydroxy-lower alkyl, lower alkanoyloxy-lower alkyl, carboxy-lower alkyl, lower alkoxycarbonyl-lower alkyl, cyano- or amino-substituted lower alkoxycarbonyl-lower alkyl, carbamoyl-lower alkyl, carbamoyloxy-lower alkyl, cyano-lower alkyl, halo-lower alkyl, amino-lower alkyl, lower alkylamino-lower alkyl, di-lower alkylamino-lower alkyl, lower alkanoylamino-lower alkyl, amino-carboxy-lower alkyl, sulpho-lower alkyl, amino, lower alkylamino, di-lower alkylamino, lower alkanoylamino, carboxy, lower alkoxycarbonyl, cyano- or amino-substituted lower alkoxycarbonyl, carbamoyl, cyano, sulpho, sulphamoyl, oxido and/or by optionally lower alkyl-, lower alkoxy- and/or halosubstituted phenyl, wherein radicals designated "lower" contain from 1 to 7 carbon atoms, or a salt of a compound of the formula I, characterised in that an ylide compound of the formula

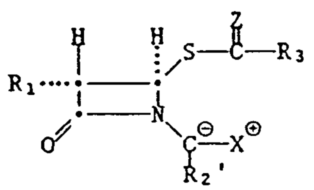

(II),

in which $R_1$ and $R_3$ have the meanings given under formula I, wherein the hydroxy group in the radical $R_1$ and other functional groups contained in the radical $R_3$ are protected by conventional protecting groups, $R_2'$ represents a protected carboxy group, Z represents oxygen or sulphur and $X^{\oplus}$ represents either a trisubstituted phosphonio group or a diesterified phosphono group together with a cation, is cyclised, or a compound of the formula

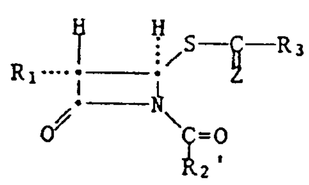

(III),

in which $R_1$ and $R_3$ have the meanings given under formula I and $R_2'$ and Z have the meanings given under formula II, wherein the hydroxy group in the radical $R_1$ and other functional groups contained in the radical $R_3$ are protected by conventional protecting groups, is treated with an organic compound of trivalent phosphorus, and in an obtainable compound protected functional groups are converted into the free functional groups, and, if desired, in an obtainable compound of the formula I a free carboxy group $R_2$ is converted into an esterified carboxy group $R_2$ that can be cleaved under physiological conditions, and/or, if desired, in an obtainable compound of the formula I a radical $R_3$ is converted into a different radical $R_3$, and/or, if desired, an obtainable compound having a salt-forming group is converted into a salt, or an obtainable salt is converted into the free

compound or into a different salt.

2. A process for the manufacture of a compound of the formula I according to claim 1 in which $R_1$ represents hydroxymethyl or 1-hydroxyethyl, $R_2$ represents carboxy or esterified carboxy that can be cleaved under physiological conditions, and $R_3$ represents 3-pyridyl or 3-pyridyl substituted by hydroxy, lower alkoxy, amino-lower alkoxy, halogen, lower alkyl, hydroxy-lower alkyl, carboxy-lower alkyl, lower alkoxycarbonyl-lower alkyl, carbamoyloxy-lower alkyl, carbamoyl-lower alkyl, amino-lower alkyl, cyano-lower alkyl, amino, carboxy, lower alkoxycarbonyl or by carbamoyl, wherein radicals designated "lower" contain from 1 to 7 carbon atoms, or a salt of a compound of the formula I.

3. A process for the manufacture of a compound of the formula I according to claim 1 in which $R_1$ represents hydroxymethyl or 1-hydroxyethyl, $R_2$ represents carboxy or esterified carboxy that can be cleaved under physiological conditions, and $R_3$ represents 3-pyridyl or 3-pyridyl substituted by lower alkyl or by halogen, wherein radicals designated "lower" contain from 1 to 7 carbon atoms, or a salt of a compound of the formula I.

4. A process for the manufacture of a compound of the formula I according to claim 1 in which $R_1$ represents (1R)-1-hydroxyethyl, and $R_2$ and $R_3$ have the meanings given in claim 1, or a salt thereof.

5. A process according to claim 1 for the manufacture of (5R,6S)-2-(3-pyridyl)-6-[(1R)-1-hydroxyethyl]-2-penem-3-carboxylic acid or a pharmaceutically acceptable salt thereof.

6. A process according to claim 1 for the manufacture of (5R,6S)-2-(6-methylpyrid-3-yl)-6-[(1R)-1-hydroxyethyl]-2-penem-3-carboxylic acid or a pharmaceutically acceptable salt thereof.

7. A process according to claim 1 for the manufacture of (5R,6S)-2-(2-chloropyrid-3-yl)-6-[(1R)-1-hydroxyethyl]-2-penem-3-carboxylic acid or a pharmaceutically acceptable salt thereof.

8. A process according to claim 1 for the manufacture of (5R,6S)-2-(6-aminopyrid-3-yl)-6-[(1R)-1-hydroxyethyl]-2-penem-3-carboxylic acid or a pharmaceutically acceptable salt thereof.

9. A process according to claim 1 for the manufacture of an ester of (5R,6S)-2-(3-pyridyl)-6-[(1R)-1-hydroxyethyl]-2-penem-3-carboxylic acid that can be cleaved under physiological conditions.

10. A process according to claim 1 for the manufacture of (5R,6S)-2-(3-pyridyl)-6-[(1R)-1-hydroxyethyl]-2-penem-3-carboxylic acid 1-ethoxycarbonyloxyethyl ester.

11. A process according to claim 1 for the manufacture of (5R,6S)-2-(3-pyridyl)-6-[(1R)-1-hydroxyethyl]-2-penem-3-carboxylic acid acetoxymethyl ester.

12. A process according to claim 1 for the manufacture of (5R,6S)-2-(3-pyridyl)-6-[(1R)-1-hydroxyethyl]-2-penem-3-carboxylic acid pivaloyloxymethyl ester.

13. A process for the manufacture of a pharmaceutical preparation, characterised in that a compound of the formula I obtainable by the process according to claim 1 or a pharmaceutically acceptable salt of such a compound is mixed with a pharmaceutically acceptable carrier.

## Revendications

### Revendications pour les Etats contractants suivants : BE, CH, DE, FR, GB, IT, LI, LU, NL, SE

1. Composés de formule

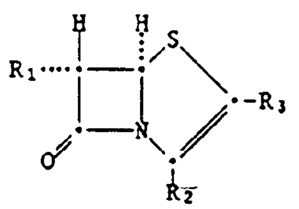

(I),

où $R_1$ est un hydroxyméthyle ou un 1-hydroxyéthyle, $R_2$ est un carboxyle ou un carboxyle estérifié séparable dans les conditions physiologiques ; et $R_3$ est un 3-pyridyle ou un 3-pyridyle substitué par un hydroxy, un alcoxy inférieur, un amino-alcoxy inférieur, un alcanoyloxy inférieur, un halogène, un mercapto, un alcoyle inférieur-thio, un aminoalcoyle inférieur-thio, un phénylthio, un alcoyle inférieur, un hydroxyalcoyle inférieur, un alcanoyloxy inférieur alcoyle inférieur, un carboxyalcoyle inférieur, un alcoxy inférieur-carbonylalcoyle inférieur, un alcoxy inférieur carbonylalcoyle inférieur substitué par un cyano ou un amino, un carbamoylalcoyle inférieur, un carbamoyloxyalcoyle inférieur, un cyanoalcoyle inférieur, un halogènalcoyle inférieur, un aminoalcoyle inférieur, un alcoyle inférieur aminoalcoyle inférieur, un dialcoyle inférieur-aminoalcoyle inférieur, un alcanoyle inférieur-aminoalcoyle inférieur, un aminocarboxyalcoyle inférieur, un sulfoalcoyle inférieur, un amino, un alcoyle

inférieur amino, un dialcoyle inférieur amino, un alcanoyle inférieur amino, un carboxyle, un alcoxy inférieur-carbonyle, un alcoxy inférieur carbonyle substitué par un cyano ou un amino, un carbamoyle, un cyano, un sulfo, un sulfamoyle, un oxydo, et/ou un phényle éventuellement substitué par un alcoyle inférieur, un alcoxy inférieur et/ou un halogène, où les radicaux décrits comme "inférieurs" contiennent de 1 à 7 atomes de carbone, et les sels des composés de formule I.

2. Composés de formule I selon la revendication 1, où $R_1$ est un hydroxyméthyle ou un 1-hydroxyéthyle, $R_2$ est un carboxy ou un carboxy estérifié séparable dans les conditions physiologiques et $R_3$ est un 3-pyridyle ou un 3-pyridyle substitué par un hydroxy, un alcoxy inférieur, un amino-alcoxy inférieur, un halogène, un alcoyle inférieur, un hydroxyalcoyle inférieur, un carboxyalcoyle inférieur, un alcoxy inférieur carbonylalcoyle inférieur, un carbamoyloxyalcoyle inférieur, un carbamoylalacoyle inférieur, un aminoalcoyle inférieur, un cyanoalcoyle inférieur, un amino, un carboxy, un alcoxy inférieur-carbonyle ou un carbamoyle ; les radicaux décrits comme "inférieurs" contenant de 1 à 7 atomes de carbone, et les sels des composés de formule I.

3. Composés de formule I selon la revendication 1, où $R_1$ est un hydroxyméthyle ou un 1-hydroxyéthyle, $R_2$ est un carboxyle ou un carboxyle estérifié séparable dans les conditions physiologiques et $R_3$ représente un 3-pyridyle ou un 3-pyridyle substitué par un alcoyle inférieur ou un halogène, où les radicaux décrits comme "inférieurs" contiennent de 1 à 7 atomes de carbone, et les sels des composés de formule I.

4. Composés de formule I selon la revendication 1, où $R_1$ est un (1R)-1-hydroxyéthyle et $R_2$ et $R_3$ ont les significations indiquées dans la revendication 1, et les sels des composés de formule I.

5. Sels pharmaceutiquement acceptables de composés de formule I selon la revendication 1.

6. Acide (5R,6S)-2-(3-pyridyl)-6-[(1R)-1-hydroxyéthyl]-2-penem-3-carboxylique et ses sels pharmaceutiquement acceptables selon la revendication 1.

7. Acide (5R,6S)-2-(6-méthyl-pyrid-3-yl)-6-[([1R)-1-hydroxyéthyl]-2-penem-3-carboxylique et ses sels pharmaceutiquement acceptables selon la revendication 1.

8. Acide (5R,6S)-2-chloropyrid-3-yl)-6-[(1R)-1-hydroxyéthyl]-2-penem-3-carboxylique et ses sels pharmaceutiquement acceptables selon la revendication 1.

9. Acide (5R,6S)-2-(6-amino-pyrid-3-yl)-[(1R)-1-hydroxyéthyl]-2-penem-3-carboxylique et ses sels pharmaceutiquement acceptables selon la revendication 1.

10. Esters séparables dans des conditions physiologiques de l'acide (5R,6S)-2-(3-pyridyl)-6-[(1R)-1-hydroxyéthyl]-2-penem-3-carboxylique selon la revendication 1

11. 1-éthoxycarbonyloxyéthylester de l'acide (5R,6S)-2-(3-pyridyl)-6-[(1R)-1-hydroxyéthyl]-2-penem-3-carboxylique selon la revendication 1.

12. Acétoxyméthylester de l'acide (5R,6S)-2-(3-pyridyl)-6-5(1R)-1-hydroxyéthyl]-2-penem-3-carboxylique selon la revendication 1.

13. Pivaloyloxyméthylester de l'acide (5R,6S)-2-(3-pyridyl)-6-[(1R)-1-hydroxyéthyl]-2-penem-3-carboxylique selon la revendication 1.

14. Composé selon la revendication 1, et ses sels pharmaceutiquement acceptables aux fins d'application dans un procédé de traitement thérapeutique du corps humain et animal.

15. Préparation pharmaceutique contenant un composé de formule I selon la revendication 1, ou un sel pharmaceutiquement acceptable d'un tel composé avec un groupe salificateur.

16. Application de composés de formule I selon la revendication 1, et de sels pharmaceutiquement acceptables de tels composés avec des groupes salificateurs, à la préparation de préparations pharmaceutiques.

17. Procédé de préparation de composés de formule I selon la revendication 1, caractérisé en ce qu'on cyclise un composé ylide de formule

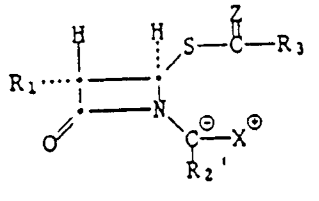

(II),

où $R_1$ et $R_3$ ont les significations données sous la formule I, où le groupe hydroxy dans le radical $R_1$ ainsi que les autres groupes fonctionnels contenus dans le radical $R_3$ sont protégés par des groupes protecteurs classiques, et $R_2'$ représente un groupe carboxyle protégé, Z un atome d'oxygène ou de soufre et $X^{\oplus}$ représente soit un groupe phosphonio trisubstitué soit un groupe phosphonio biestérifié, avec un cation, ou en ce qu'on traite un composé de formule

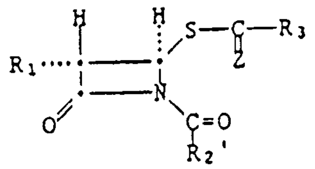

(III),

où $R_1$ et $R_3$ ont les significations données sous la formule I et $R_2'$ et Z ont les significations données sous la formule II, où le groupe hydroxy dans le radical $R_1$ ainsi que les autres groupes fonctionnels contenus dans le radical $R_3$ sont protégés par des groupes protecteurs classiques, avec un composé organique du phosphore trivalent, et en ce que dans un composé obtenu on transforme les groupes fonctionnels protégés en les groupes fonctionnels libres et, si on le désire, en ce que dans un composé de formule I obtenu, on transforme un groupe carboxyle libre $R_2$ en un groupe carboxyle estérifié séparable dans les conditions physiologiques $R_2$ et/ou, si on le désire, en ce que dans un composé obtenu de formule I on transforme le radical $R_3$ en un autre radical $R_3$ et/ou, si on le désire, en ce qu'on transforme un composé obtenu à groupe salificateur en un sel ou un sel obtenu en le composé libre ou en un autre sel.

18. Les composés que l'on peut obtenir selon le procédé de la revendication 17.

**Revendications pour les Etats contractants suivants : AT, ES, GR**

1. Procédé de préparation de composés de formule

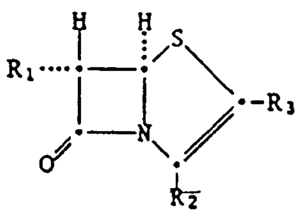

(I),

où $R_1$ est un hydroxyméthyle ou un 1-hydroxyéthyle, $R_2$ est un carboxyle ou un carboxyle estérifié séparable dans les conditions physiologiques ; et $R_3$ est un 3-pyridyle ou un 3-pyridyle substitué par un hydroxy, un alcoxy inférieur, un amino-alcoxy inférieur, un alcanoyloxy inférieur, un halogène, un mercapto, un alcoyle inférieur-thio, un aminoalcoyle inférieur-thio, un phénylthio, un alcoyle inférieur, un hydroxyalcoyle inférieur, un alcanoyloxy inférieur alcoyle inférieur, un carboxyalcoyle inférieur, un alcoxy inférieurcarbonylalcoyle inférieur, un alcoxy inférieurcarbonylalcoyle inférieur substiitué par un cyano ou un amino, un carbamoylalcoyle inférieur, un carbamoyloxyalcoyle inférieur, un cyanoalcoyle inférieur, un halogènalcoyle inférieur, un aminoalcoyle inférieur, aun alcoyle inférieur aminoalcoyle inférieur, un dialcoyle inférieur-amino-alcoyle inférieur, un alcanoyle inférieur-aminoalcoyle inférieur, un aminocarboxyalcoyle inférieur, un sulfoalcoyle inférieur, un amino, un alcoyle inférieur amino, un dialcoyle inférieur amino, un alcanloyle inférieur amino, un carboxyle, un alcoxy inférieurcarbonyle, un alcoxy inférieur carbonyle substitué par un cyano ou un amino, un carbamoyle, un cyano, un sulfo, un sulfamoyle, un oxydo, et/ou un phényle éventuellement substitué par un alcoyle inférieur, un alcoxy inférieur et/ou un halogène, où les raidcaux décrits comme "inférieurs" contiennent de 1 à è atomes de carbone, et des sels des composés de formule I

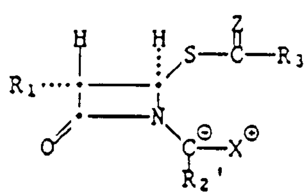

(II),

, caractérisé en ce que $R_1$ et $R_3$ ont les significations données sous la formule I, où le groupe hydroxy dans le radical $R_1$ ainsi que les autres groupes fonctionnels contenus dans le radical $R_3$ sont protégés par des groupes protecteurs classiques, et $R_2'$ représente un groupe carboxyle protégé, Z un atome d'oxygène ou de soufre et $X_O$ représente soit un groupe phosphonio trisubstitué soit un groupe phosphonio biestérifié, avec un cation, ou en ce qu'on traite un composé de formule

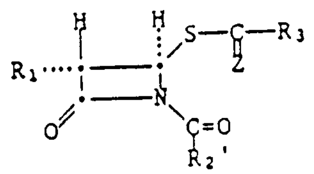

(III),

où $R_1$ et $R_3$ ont les significations données sous la formule I et $R_2'$ et Z ont les significations données sous la formule II, où le groupe hydroxy dans le radical $R_1$ ainsi que les autres groupes fonctionnels contenus dans le radical $R_3$ sont protégés par des groupes protecteurs classiques, avec un composé organique du phosphore trivalent, et en ce que dans un composé obtenu on transforme les groupes fonctionnels protégés en les groupes fonctionnels libres et, si on le désire, en ce que dans un composé de formule I obtenu, on transforme un groupe carboxyle libre $R_2$ en un groupe carboxyle estérifié séparable dans les conditions physiologiques $R_2$ et/ou, si on le désire, en ce que dans un composé obtenu de formule I on transforme les radical $R_3$ en un autre radical $R_3$ et/ou, si on le désire, en ce qu'on transforme un composé obtenu à groupe salificateur en un sel ou un sel obtenu en le composé libre ou en un autre sel.

2. Procédé de préparation de composés de formule I selon la revendication 1, où $R_1$ est un hydroxyméthyle ou un 1-hydroxyéthyle, $R_2$ représente un carboxyle ou un carboxyle estérifié séparable dans les conditions physiologiques et $R_3$ représente un 3-pyridyle ou un 3-pyridyle substitué par un hydroxy, alcoxy inférieur, amino-alcoxy inférieur, halogène, alcoyle inférieur, hydroxyalcoyle inférieur, carboxyalcoyle inférieur, alcoxy inférieur carbonylalcoyle inférieur, carbamoyloxyalcoyle inférieur, carbamoylalcoyle inférieur, aminoalcoyle inférieur, cyanoalcoyle inférieur, amino, carboxy, alcoxy inférieur carbonyle ou carbamoyle, où les radicaux décrits comme "inférieurs" contiennent de 1 à 7 atomes de carbone, et des sels des composés de formule I.

3. Procédé de préparation de composés de formule I selon la revendication 1, où $R_1$ est un hydroxyméthyle ou un 1-hydroxyéthyle, $R_2$ est un carboxyle ou un carboxyle estérifié séparable dans les conditions physiologiques et $R_3$ représente un 3-pyridyle ou un 3-pyridyle substitué par un alcoyle inférieur ou un halogène, où les radicaux décrits comme "inférieurs" contiennent de 1 à 7 atomes de carbone, et des sels des composés de formule I.

4. Procédé de préparation de composés de formule I selon la revendication 1, où $R_1$ est un (1R)-1-hydroxyéthyle et $R_2$ et $R_3$ ont les significations données dans la revendication 1, et de leurs sels.

5. Procédé selon la revendication 1, de préparation de l'acide (5R,6S)-2-(3-pyridyl)-6-[(1R)-1-hydroxyéthyl]-2-penem-3-carboxylique et de ses sels pharmaceutiquement acceptables.

6. Procédé selon la revendication 1 de préparation de l'acide (5R,6S)-2-(6-méthyl-pyrid-3-yl)-6-[(1R)-1-hydroxyéthyl]-2-penem-3-carboxylique et de ses sels pharmaceutiquement acceptables.

7. Procédé selon la revendication 1 de préparation de l'acide (5R,6S)-2-(2-chloropyrid-3-yl)-6-[(1R)-1-hydroxyéthyl]-2-penem-3-carboxylique et de sels pharmaceutiquement acceptables.

8. Procédé selon la revendication 1 de préparation de l'acide (5R,6S)-2-(6-amino-pyrid-3-yl)-6-[1R)-1-hydroxyéthyl]-2-penem-3-carboxylique et de ses sels pharmaceutiquement acceptables.

9. Procédé selon la revendication 1 de préparation d'esters séparables dans les conditions physiologiques de l'acide (5R,6S)-2-(3-pyridyl)-6-[(1R)-1-hydroxyéthyl]-2-penem-3-carboxylique.

10. Procédé selon la revendication 1, de préparation du 1-éthoxycarbonyloxyéthylester de l'acide (5R,6S)-2-(3-pyridyl)-6-[(1R)-1-hydroxyéthyl]-2-penem-3-carboxylique.

11. Procédé selon la revendication 1, de préparation de l'acétoxyméthylester de l'acide (5R,6S)-2-(3-pyridyl)-6-[(1R)-1-hydroxyéthyl]-2-penem-3-carboxylique.

12. Procédé selon la revendication 1, de préparation du pivaloyloxyméthylester de l'acide (5R,6S)-2-(3-pyridyl)-6[(1R)-1-hydroxyéthyl]-2-penem-3-carboxylique.

13. Procédé de préparation de préparations pharmaceutiques, caractérisé en ce qu'on mélange un composé de formule I que l'on peut obtenir selon le procédé de la revendication 1 ou un sel pharmaceutiquement acceptable d'un tel composé avec un support pharmaceutiquement acceptable.